(19) Europäisches Patentamt
European Patent Office
Office européen des brevets

(11) Veröffentlichungsnummer: **0 541 042 A1**

# (12) EUROPÄISCHE PATENTANMELDUNG

(21) Anmeldenummer: **92118814.0**

(22) Anmeldetag: **03.11.92**

(51) Int. Cl.5: **C07D 213/81**, C07D 213/82, A61K 31/44

(30) Priorität: **05.11.91 DE 4136380**

(43) Veröffentlichungstag der Anmeldung:
**12.05.93 Patentblatt 93/19**

(84) Benannte Vertragsstaaten:
**AT BE CH DE DK ES FR GB GR IT LI LU MC NL PT SE**

(71) Anmelder: **HOECHST AKTIENGESELLSCHAFT**
**Postfach 80 03 20**
**W-6230 Frankfurt am Main 80(DE)**

(72) Erfinder: **Weidmann, Klaus, Dr.**
**Scheibenbuschweg 2**
**W-6242 Kronberg/Ts.(DE)**
Erfinder: **Bickel, Martin, Dr.**
**Mittelstedter Weg 3**
**W-6380 bad Homburg(DE)**
Erfinder: **Günzler-Pukall, Volkmar, Dr.**
**Gross-Seelheimer Strasse 13**
**W-3550 Marburg(DE)**
Erfinder: **Schubert, Gerrit, Dr.**
**Feldbergstrasse 2a**
**W-6233 Kelkheim/Ts.(DE)**

(54) **Pyridin-2,4- und 2,5-dicarbonsäureamide und deren Derivate, Verfahren zu ihrer Herstellung sowie deren Verwendung als Arzneimittel.**

(57) Es werden Pyridin -2,4- und 2,5-dicarbonsäureamide der Formel (I)

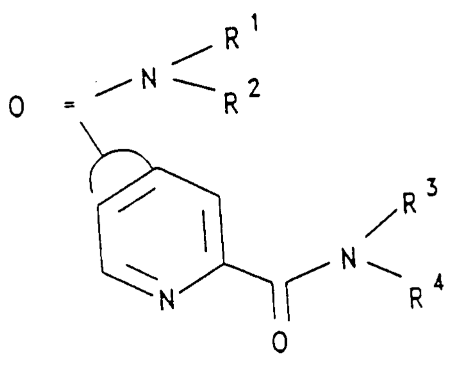

(I)

beschrieben, in der $R^1$, $R^2$, $R^3$ und $R^4$ die angegebenen Bedeutungen haben, sowie ihre Verwendung als Arzneimittel, insbesondere als Fibrosuppressiva und Immunsuppressiva.

EP 0 541 042 A1

Verbindungen, die die Enzyme Prolin- und Lysinhydroxylase inhibieren, bewirken eine sehr selektive Hemmung der Kollagenbiosynthese durch Beeinflussung der kollagenspezifischen Hydrorylierungsreaktionen. In deren Verlauf wird proteingebundenes Prolin oder Lysin durch die Enzyme Prolin- bzw. Lysinhydroxylase hydroxyliert. Wird diese Reaktion durch Inhibitoren unterbunden, so entsteht ein nicht funktionsfähiges, unterhydroxyliertes Kollagenmolekül, das von den Zellen nur in geringer Menge in den extrazellulären Raum abgegeben werden kann. Das unterhydroxylierte Kollagen kann außerdem nicht in die Kollagenmatrix eingebaut werden und wird sehr leicht proteolytisch abgebaut. Als Folge dieser Effekte verringert sich nsgesamt die Menge des extrazellulär abgelagerten Kollagens.

Es ist bekannt, daß die Inhibierung der Prolinhydroxylase durch bekannte Inhibitoren wie $\alpha,\alpha'$-Dipyridyl zu einer Hemmung der $Cl_q$-Biosynthese von Makrophagen führt (W Müller et al., FEBS Lett. 90 (1978), 218; Immunbiology 155 (1978), 47). Dadurch kommt es zu einem Ausfall des klassischen Weges der Komplementaktivierung. Inhibitoren der Prolinhydroxylase wirken daher auch als Immunsuppressiva, z. B. bei Immunkomplexkrankheiten.

Es ist bekannt, daß das Enzym Prolinhydroxylase durch Pyridin-2,4- und -2,5-dicarbonsäure effektiv gehemmt wird (K. Majamaa et al., Eur. J. Biochem. 138 (1984) 239-245). Diese Verbindungen sind in der Zellkultur allerdings nur in sehr hohen Konzentrationer als Hemmstoffe wirksam (Tschank, G. et al., Biochem. J. 238 (1987) 625-633).

In der DE-A 34 32 094 werden Pyridin-2,4- und -2,5-dicarbonsäurediester mit 1 - 6 C-Atomen im Esteralkylteil als Arzneimittel zur Inhibierung der Prolin- und Lysinhydroxylase beschrieben.

Diese niedrig-alkylierten Diester haben jedoch den Nachteil, daß sie zu schnell im Organismus zu den Säuren gespalten werden und nicht in genügend hoher Konzentration an ihren Wirkort in der Zelle gelangen und damit für eine eventuelle Verabreichung als Arzneimittel weniger geeignet sind.

Die DE-A 37 03 959, DE-A 37 03 962 und DE-A 37 03 963 beschreiben in allgemeiner Form gemischte Ester/Amide, höher alkylierte Diester und Diamide der Pyridin-2,4- und -2,5-dicarbonsäure, die Kollagenbiosynthese im Tiermodell wirksam hemmen.

So wird in der DE-A 37 03 959 unter anderem die Synthese von N,N'-Bis(2-methoxyethyl)-pyridin-2,4-dicarbonsäurediamid und N,N'-Bis(3-isopropoxypropyl)-pyridin-2,4-dicarbonsäurediamid beschrieben.

In den deutschen Patentanmeldungen DE-A-38 26 471 und DE-A-38 28 140 wird ein verbessertes Verfahren zur Herstellung von N,N'-Bis(2-methoxyethyl)-pyridin-2,4-dicarbonsäurediam d vorgeschlagen. Die deutsche Patentanmeldung DE-A-39 24 093 schlägt neue N,N'-Bis(alkoxyalkyl)-pyridin-2,4-dicarbonsäurediamide vor.

Es bestand die Notwendigkeit nach anderen Verbindungen zu suchen, die eine verbesserte pharmakologische Wirksamkeit hinsichtlich der Inhibierung der Lysin-und Prolinhydroxylase aufweisen.

Überraschenderweise wurde nun gefunden, daß die folgenden Pyridin -2,4-und - 2,5-dicarbonsäurediamide die gestellte Aufgabe erfüllen.

Gegenstand der Erfindung sind daher Verbindungen der allgemeinen Formel I

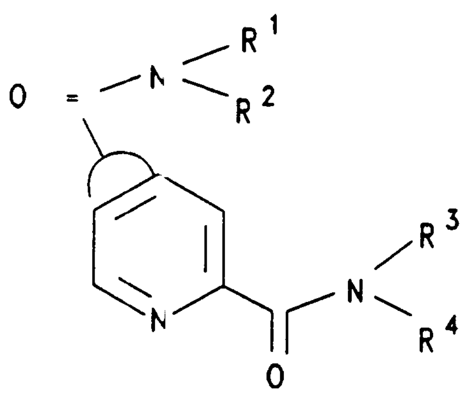

(I)

worin

$R^1$, $R^2$, $R^3$ und $R^4$ gleich oder verschieden sind und

A einen verzweigten oder unverzweigten, aliphatischen oder cycloaliphatischen $(C_1-C_{12})$-Alkylrest, $(C_1-C_{12})$-Alkenylrest oder einen $(C_1-C_{12})$-Alkinylrest bedeutet, der einfach oder mehrfach vorzugsweise einfach oder zweifach substituiert ist mit

einem $(C_1-C_8)$-Alkoxycarbonyloxy, $(C_1-C_8)$-Alkoxy-$(C_1-C_8)$-alkoxycarbonyloxy, $(C_6-C_{12})$-Ary-

loxycarbonyloxy, $(C_7-C_{11})$-Aralkyloxycarbonyloxy, $(C_7-C_{11})$-Aralkylcarbonyloxy, Cinnamoyl, Cinnamoyloxy, $(C_6-C_{12})$-Arylcarbonyloxy, $(C_3-C_8)$-Alkenylcarbonyloxy, $(C_3-C_8)$-Alkinylcarbonyloxy, $(C_3-C_8)$-Cycloalkylcarbonyloxy, $(C_1-C_{12})$-Alkoxy-$(C_1-C_{12})$-alkoxy, $(C_1-C_{12})$-Alkoxy-amino, $(C_1-C_{12})$-Alkoxy-N $(C_1-C_6)$-alkylamino, $(C_1-C_{12})$-Alkoxy-N,N $(C_1-C_6)$-dialkylamino, Carbamoyloxy, N-$(C_1-C_8)$-Alkylcarbamoyloxy, N,N-Di-$(C_1-C_8)$-alkylcarbamoyl, N-$(C_3-C_8)$-Cycloalkylcarbamoyl, N-$(C_6-C_{12})$-Arylamino, N-$(C_7-C_{11})$-Aralkylamino, N-Alkyl-Aralkylamino, N-Alkyl-Arylamino, ( $C_3-C_8$)-Cycloalkanoylamino, $(C_1-C_8)$-Alkanoylamino, $(C_6-C_{12})$-Aroylamino, $(C_7-C_{11})$-Aralkanoylamino, $(C_1-C_8)$-Alkanoyl-$(C_1-C_8)$-alkylamino, $(C_3-C_8)$-Cycloalkanoyl-$(C_1-C_8)$-alkylamino,$(C_6-C_{12})$-Aroyl-$(C_1-C_8)$alkylamino, $(C_7-C_{11})$-Aralkanoyl-$(C_1-C_8)$-alkylamino,$(C_1-C_8)$-Alkylmercapto, $(C_1-C_8)$-Alkylsulfinyl, $(C_1-C_8)$-Alkylsulfonyl, $(C_1-C_8)$-Alkylcarbonyl,$(C_3-C_8)$-Cycloalkylcarbonyl, Nitro, Trifluormethyl, Phenylmercapto, Phenylsulfonyl, Phenylsulfinyl, Sulfamoyl, N-$(C_1-C_6)$-Alkylsulfamoyl, N,N-Di-$(C_1-C_6)$-alkylsulfamoyl, $(C_1-C_8)$-Alkyl-sulfonamido, Arylsulfonamido, wobei die in vorstehenden Substituenten vorhandenen Aryl- und Aralkylreste auch heterocyclischer Natur sein können und/oder, wie auch Alkyl, mit 1, 2, 3, 4 oder 5 gleichen oder verschiedenen Substituenten aus der Reihe Halogen, Cyano, Nitro, Trifluormethyl, $(C_1-C_6)$-Alkyl Hydroxy, $(C_1-C_6)$-Hydroxyalkyl, $(C_1-C_6)$-Alkoxy, $-O-[CH_2-]_xC_fH_{(2f+1-g)}F_g$, $-OCF_2Cl$, $-O-CF_2-CHFCl$, Trifluormethyl $(C_1-C_6)$-Alkylmercapto, $(C_1-C_6)$-Alkylsulfinyl, $(C_1-C_6)$-Alkylsulfonyl, $(C_1-C_6)$-Alkylcarbonyl, $(C_1-C_6)$-Alkoxycarbonyl, Carbamoyl, N-$(C_1-C_4)$-Alkylcarbamoyl, N,N-Di-$(C_1-C_4)$-alkylcarbamoyl, $(C_1-C_6)$-Alkylcarbonyloxy, $(C_3-C_8)$-Cycloalkyl, Phenyl, Benzyl, Phenoxy, Benzyloxy, NR'-R'', Phenylmercapto, Phenylsulfonyl, Phenylsulfinyl, Sulfamoyl, N-$(C_1-C_4)$-Alkylsulfamoyl oder N'N-Di-$(C_1-C_4)$-alkylsulfamoyl, insbesondere bis zu mit 3 der vorstehend genannten gleichen oder verschiedenen Substituenten substituiert sind und eine $CH_2$-Gruppe der Alkylkette gegeberenfalls durch O, S, SO, $SO_2$ oder NR' ersetzt ist, oder mit

einem substituierten $(C_6-C_{12})$-Arylrest oder Heteroarylrest, der 1, 2, 3, 4 oder 5 gleiche oder verschiedene Substituenten aus der Reihe Hydroxy, Trifluormethyl, $(C_1-C_6)$-Hydroxyalkyl, $-O-[CH_2-]_xC_fH_{(2f+1-g)}F_g$, $-OCF_2Cl$, $-OCF_2-CHFCl$, $(C_1-C_6)$-Alkylmercapto, $(C_1-C_6)$-Alkylsulfinyl, $(C_1-C_6)$-Alkylsulfonyl, $(C_1-C_6)$-Alkylcarbonyl, $(C_1-C_6)$-Alkoxycarbonyl, Carbamoyl, N-$(C_1-C_4)$-Alkylcarbamoyl, N,N-Di-$(C_1-C_4)$-alkylcarbamoyl, $(C_1-C_6)$-Alkylcarbonyloxy, $(C_3-C_8)$-Cycloalkyl, Phenyl, Benzyl, Phenoxy, Benzyloxy, NR'-R'', Phenylmercapto, Phenylsulfonyl, Phenylsulfinyl, Sulfamoyl, N-$(C_1-C_4)$-Alkylsulfamoyl, N,N-Di-$(C_1-C_4)$-alkylsulfamoyl, $(C_1-C_8)$-Alkoxycarbonyloxy, $(C_1-C_8)$-Alkoxy-$(C_1-C_8)$-alkoxycarbonyloxy, $(C_6-C_{12})$-Aryloxycarbonyloxy, $(C_7-C_{11})$-Aralkyloxycarbonyloxy, $(C_7-C_{11})$-Aralkylcarbonyloxy, Cinnamoyl, Cinnamoyloxy, $(C_6-C_{12})$-Arylcarbonyloxy, $(C_3-C_8)$-Alkenylcarbonyloxy, $(C_3-C_8)$-Alkinylcarbonyloxy, $(C_3-C_8)$-Cycloalkylcarbonyloxy, $(C_1-C_{12})$-Alkoxy-$(C_1-C_{12})$-alkoxy, $(C_1-C_{12})$-Alkoxy-amino, $(C_1-C_{12})$-Alkoxy-N $(C_1-C_6)$-alkylamino, $(C_1-C_{12})$-Alkoxy-N,N$(C_1-C_6)$-dialkylamino, Carbamoyloxy, N-$(C_1-C_8)$-Alkylcarbamoyloxy, N,N-Di-$(C_1-C_8)$-alkylcarbamoyl, N-$(C_3-C_8)$-Cycloalkylcarbamoyl, N-$(C_6-C_{12})$-Arylamino, N-$(C_7-C_{11})$-Aralkylamino, N-Alkyl-Aralkylamino, N-Alkyl-Arylamino, $(C_3-C_8)$-Cycloalkanoylamino, $(C_1-C_8)$-Alkanoylamino, $(C_6-C_{12})$-Aroylamino, $(C_7-C_{11})$-Aralkanoylamino, $(C_1-C_8)$-Alkanoyl-$(C_1-C_8)$-alkylamino, $(C_3-C_8)$-Cycloalkanoyl-$(C_1-C_8)$-alkylamino,$(C_6-C_{12})$-Aroyl-$(C_1-C_8)$-alkylamino, $(C_7-C_{11})$-Aralkanoyl-$(C_1-C_8)$-alkylamino,$(C_1-C_8)$-Alkylmercapto, $(C_1-C_8)$-Alkylsulfinyl, $(C_1-C_8)$-Alkylsulfonyl, $(C_1-C_8)$-Alkylcarbonyl,$(C_3-C_8)$Cycloalkylcarbonyl, Nitro, Trifluormethyl, Phenylmercapto, Phenylsulfonyl, Phenylsulfinyl, Sulfamoyl, N-$(C_1-C_6)$-Alkylsulfamoyl, N,N-Di-$(C_1-C_6)$-alkylsulfamoyl, $(C_1-C_8)$-Alkyl-sulfonamido und Arylsulfonamido trägt, wobei die in vorstehenden Substituenten vorhandenen Aryl- und Aralkylreste auch heterocyclischer Natur sein können und/oder, wie auch Alkyl, mit 1, 2, 3, 4 oder 5 gleichen oder verschiedenen Substituenten aus der Reihe Halogen, Cyano, Nitro, Trifluormethyl, $(C_1-C_6)$-Alkyl Hydroxy, $(C_1-C_6)$-Hydroxyalkyl, $(C_1-C_6)$-Alkoxy, substituiert sein können, oder mit

einem substituierten $(C_6-C_{12})$-Aryloxyrest, $(C_7-C_{11})$-Aralkyloxyrest oder Heteroaryloxyrest, der 1, 2, 3, 4 oder 5 gleiche oder verschiedene Substituenten aus der Reihe Hydroxy, Halogen, Cyano, Nitro, Trifluormethyl, $(C_1-C_6)$-Hydroxyalkyl, $(C_1-C_6)$Alkoxy, $[CH_2-]_xC_fH_{(2f+1-g)}F_g$, $-OCF_2-CHFCl$, $(C_1-C_6)$-Alkylmercapto, $(C_1-C_6)$-Alkylsulfinyl, $(C_1-C_6)$-Alkylsulfonyl, $(C_1-C_6)$-Alkylcarbonyl, $(C_1-C_6)$-Alkoxycarbonyl, Carbamoyl, N-$(C_1-C_4)$-Alkylcarbamoyl, N,N-Di-$(C_1-C_4)$-alkylcarbamoyl, $(C_1-C_6)$-Alkylcarbonyloxy, $(C_3-C_8)$-Cycloalkyl, Carboxyl, Phenyl, Benzyl, Phenoxy, Benzyloxy, NR'-R'', Phenylmercapto, Phenylsulfonyl, Phenylsulfinyl, Sulfamoyl, N-$(C_1-C_4)$-Alkylsulfamoyl, N,N-Di-$(C_1-C_4)$-alkylsulfamoyl Aminoalkyl, N-$(C_1-C_8)$alkylamino-$(C_1-C_{12})$-alkyl oder N-Di-$(C_1-C_8)$-alkylamino-$(C_1-C_{12})$-alkyl trägt, gegebenenfalls bis zu mit 3 der vorstehend genannten gleichen oder verschiedenen Substituenten substituiert ist und eine $CH_2$-Gruppe der Alkylkette gegebenenfalls durch O, S, SO, $SO_2$ oder NR' ersetzt ist, oder mit

einem Rest der allgemeinen Formel II

$- O - R^5$ (II),

worin

$R^5$ eine über ihren Acylrest gebundene Aminosäure, ihr Derivat oder eine Alkoholschutzgruppe bedeutet,

B einen substituierten $(C_6-C_{12})$Arylrest, $(C_7-C_{11})$Aralkylrest oder einen Heteroarylrest bedeutet, der einfach oder mehrfach, vorzugsweise ein- oder zweifach substituiert ist mit

Hydroxy, Amino, $(C_1-C_8)$-Alkoxycarbonyl, $(C_1-C_8)$-Alkylcarbonyloxy, $(C_1-C_8)$-Alkylamino, Di-$(C_1-C_8)$-alkylamino, $(C_1-C_6)$-Hydroryalkyl, $-O-[CH_2-]_xC_fH_{(2f+1-g)}F_g$, $-OCF_2Cl$, $-OCF_2-CHFCl$, Carbamoyl, N-$(C_1-C_8)$-Alkylcarbamoyl, N,N-Di-$(C_1-C_8)$-alkylcarbamoyl, $(C_1-C_8)$-Alkylcarbonyloxy, $(C_3-C_8)$-Cycloalkyl, Phenyl, Benzyl, Phenoxy, Benzyloxy, Aminoalkyl, N-$(C_1-C_8)$-alkylamino $(C_1-C_{12})$-alkyl oder N,N-Di-$(C_1-C_8)$-alkylamino-$(C_1-C_{12})$-alkyl, $(C_1-C_8)$-Alkoxycarbonyloxy, $(C_1-C_8)$-Alkoxy-$(C_1-C_8)$-alkoxycarbonyloxy, $(C_6-C_{12})$-Aryloxycarbonyloxy, $(C_7-C_{11})$-Aralkyloxycarbonyloxy, $(C_7-C_{11})$-Aralkylcarbonyloxy, Cinnamoyl, Cinnamoyloxy, $(C_6-C_{12})$-Arylcarbonyloxy, $(C_3-C_8)$-Alkenylcarbonyoxy, $(C_3-C_8)$-Alkinylcarbonyloxy, $(C_3-C_8)$-Cycloalkylcarbonyloxy, $(C_1-C_{12})$-Alkoxy-$(C_1-C_{12})$-alkoxy, $(C_1-C_{12})$-Alkoxy-amino, $(C_1-C_{12})$-Alkoxy-N $(C_1-C_6)$-alkylamino, $(C_1-C_{12})$-Alkoxy-N,N $(C_1-C_6)$-dialkylamino, Carbamoyloxy, N-$(C_1-C_8)$-Alkylcarbamoyloxy, N,N-Di-$(C_1-C_8)$-alkylcarbamoyl, N-$(C_3-C_8)$-Cycloalkylcarbamoyl, N-$(C_6-C_{12})$-Arylamino, N-$(C_7-C_{11})$-Aralkylamino, N-Alkyl-Aralkylamino, N-Alkyl-Arylamino, $(C_3-C_8)$-Cycloalkanoylamino, $(C_1-C_8)$-Alkanoylamino, $(C_6-C_{12})$-Aroylamino, $(C_7-C_{11})$-Aralkanoylamino, $(C_1-C_8)$-Alkanoyl-$(C_1-C_8)$-alkylamino, $(C_3-C_8)$-Cycloalkanoyl-$(C_1-C_8)$-alkylamino, $(C_6-C_{12})$-Aroyl-$(C_1-C_8)$-alkylamino, $(C_7-C_{11})$-Aralkanoyl-$(C_1-C_8)$-alkylamino, $(C_1-C_8)$-Alkylmercapto, $(C_1-C_8)$-Alkylsulfinyl, $(C_1-C_8)$-Alkylsulfonyl, $(C_1-C_8)$-Alkylcarbonyl, $(C_3-C_8)$-Cycloalkylcarbonyl, Nitro, Trifluormethyl, Phenylmercapto, Phenylsulfonyl, Phenylsulfinyl, Sulfamoyl, N-$(C_1-C_6)$-Alkylsulfamoyl, N,N-Di-$(C_1-C_6)$-alkylsulfamoyl, $(C_1-C_8)$-Alkyl-sulfonamido, Arylsulfonamido,

C einen substituierten $(C_1-C_{12})$Alkoxyrest, $(C_3-C_8)$-Cycloalkoxy, $(C_6-C_{12})$-Aryloxyrest oder einen $(C_7-C_{11})$-Aralkyloxyrest bedeuten, der einfach oder mehrfach, vorzugsweise ein- und zweifach substituiert ist mit

Halogen, Trifluormethyl, $(C_1-C_6)$-Alkoxy, Hydroxy, $(C_1-C_6)$-Hydroxyalkyl, NR'R'' oder Cyano,

wobei jeweils

R' und R'' gleich oder verschieden sind und Wasserstoff, $(C_6-C_{12})$-Aryl, $(C_1-C_8)$-Alkyl, $(C_1-C_8)$-Alkylcarbonyl, $(C_7-C_{11})$Aralkylcarbonyl oder $(C_6-C_{12})$-Arylcarbonyl bedeuten oder mit dem Stickstoff einen gesärtigten heterocyclischen Ring bilden, vorzugsweise einen 5- oder 6-gliedrigen Ring,

und die genannten Reste $R^1$, $R^2$, $R^3$ und $R^4$ in Kombination mit einem

$(C_1-C_{12})$-Alkylrest, der einfach oder mehrfach, vorzugsweise ein- und zweifach, mit Wasserstoff, Halogen, Hydroxy, Cyano, Amino, Carboxyl, $(C_1-C_4)$-Alkoxy, $(C_1-C_4)$-Alkoxycarbonyl, $(C_1-C_4)$-Alkylcarbonyloxy, $(C_1-C_4)$ Alkyl- oder $(C_1-C_4)$-Dialkylamino oder einem Phenylring, der ein-, zwei- oder dreifach mit den Resten Halogen, Nitro, $(C_1-C_4)$-Alkyl oder $(C_1-C_4)$-Alkoxy substituiert ist, auftreten können, oder in Kombination mit einem

Aryl- oder Heteroarylrest, der seinerseits gegebenenfalls mit Halogen, Nitro, Cyano, $(C_1-C_4)$-Alkyl oder $(C_1-C_4)$-Alkoxy 1-, 2- oder 3-fach substituiert sein kann, auftreten können, zuzüglich aller Derivate, die in ihren Amino- oder Hydroxygruppen eine entsprechende Schutzgruppe aufweisen sowie die physiologisch wirksame Salze und

n = 0 oder 1,
f = 1 bis 8, vorzugsweise 1 bis 5,
g = 0,1 bis (2f+1),
x 0, 1, 2 oder 3, vorzugsweise 0 oder 1 ist.

Unter Aryl-, Aryloxy-, Heteroaryl- bzw. Heteroaryloxyverbindungen versteht man insbesondere Phenyl- und Naphtyl- bzw. unsubstituierte 5- und 6-gliedrige heteroaromatische Ringe mit 1, 2 oder 3 Stickstoff- und/oder Sauerstoff und/oder Schwefelatomen, wie Pyridyl-, Pyridazyl-, Pyrimidyl-, Pyrazyl-, Imidazolyl-, Triazolyl-, Thienyl-, Oxazolyl-, und Thiazolyl-Derivate, und deren benzoannellierte Derivate. Unter dem Rest $(C_7-C_{11})$-Aralkyloxy ist vorzugsweise ein substituierter Phenylalkyloryrest der Formel III

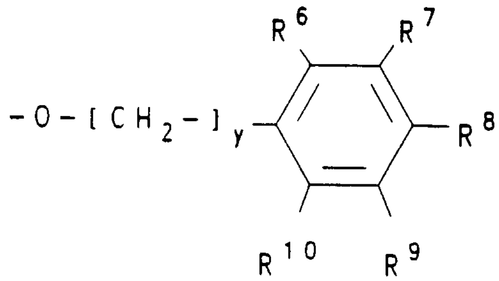

(III)

zu verstehen,
worin $R^6$, $R^7$, $R^8$ und $R^{10}$ gleich oder verschieden sind und Wasserstoff, Halogen, Cyano, Nitro, Trifluormethyl, $(C_1-C_6)$-Alkyl, $(C_1-C_6)$-Alkoxy, $-O-[CH_2-]_xC_fH_{(2f+1-g)}F_g$, $-OCF_2Cl$, $-O-CF_2-CHFCl$, $(C_1-C_6)$-Alkylmercapto, $(C_1-C_6)$-Alkylsulfinyl, $(C_1-C_6)$-Alkylsulfonyl, $(C_1-C_6)$-Alkylcarbonyl, $(C_1-C_6)$-Alkorycarbonyl, Carbamoyl, N-$(C_1-C_4)$-Alkylcarbamoyl, N,N-Di-$(C_1-C_4)$-alkylcarbamoyl, $(C_1-C_6)$-Alkylcarbonyloxy, $(C_3-C_8)$-Cycloalkyl, Phenyl, Benzyl, Phenoxy, Benzyloxy, NR'R'', wie Amino, Anilino, N-Methylanilino, Phenylmercapto, Phenylsulfonyl, Phenylsulfinyl, Sulfamoyl, N-$(C_1-C_4)$-Alkylsulfamoyl oder N,N-Di-$(C_1-C_4)$-alkylsulfamoyl bedeuten, oder zwei benachbarte Substituenten gemeinsam eine Kette $-[CH_2-]_n$ oder $-CH=CH-CH=CH-$ bedeuten, wobei eine $CH_2$-Gruppe der Kette gegebenenfalls durch O, S, SO, $SO_2$ oder NR' ersetzt ist, Y = 1, 2, 3 oder 4, vorzugsweise 0 und 1 bedeutet und die übrigen der Substituenten $R^6$, $R^7$, $R^8$, $R^9$ und $R^{10}$ wie vorstehend definiert sind.

Von den genannten Aminosäuren werden insbesondere die natürlichen $\alpha$-Aminosäuren bevorzugt.

Unter Aminoschutzgruppen versteht man insbesondere solche Gruppen, die in R. Geiger und W. König "The Peptides" Volume 3, "Protection of Functional Groups in Peptide Synthesis", E. G. Gross, J. Meienhofer Edit, Academic Press, New York (1981), insbesondere Seiten 7 - 46, beschrieben sind.

Ebenso werden solche Gruppen in A. Hubbuch, Schutzgruppen in der Peptidsynthese, Kontakte 3/79, Seiten 14-23 beschrieben.

Insbesondere bevorzugt sind folgende Aminoschutzgruppen:
Acetamidomethyl,
1-Adamantyloxycarbonyl,
1-(1-Adamantyl)-1-methyl-ethoxycarbonyl,
Allyloxycarbonyl,
tert.-Butyloxycarbonyl,
1-(4-Biphenylyl)-1-methyl-ethoxycarbonyl,
Dicyclohexylcarbodiimid,
$\alpha,\alpha$-Dimethyl-3,5-dimethoxybenzyloxycarbonyl,
4-Dihydroxyborylbenzyloxycarbonyl,
9-Fluorenylmethyloxycarbonyl,
1-Hydroxybenzotriazol
3-Hydroxy-4-oxo-3,4-dihydro-1,2,3-benzotriazin,
Isobornyloxycarbonyl,
1-Methyl-cyclobutyloxycarbonyl,
4-Methoxybenzyloxycarbonyl,
Methylsulfonylethyloxycarbonyl,
4-Pyridylmethyloxycarbonyl,
2,2,2-Trichloro-tert.-butyloxycarbonyl,
Benzyloxycarbonyl,
halogensubstituiertes Benzyloxycarbonyl,
4-Nitro-benzyloxycarbonyl,
2-Phosphonoethyloxycarbonyl,
Phenylsulfonylethoxycarbonyl,
Toluolsulfonylethoxycarbonyl,
2,3,5-Trimethyl-4-methoxy-phenylsulfonyl,
Benzotriazol-1-yl-oxy-tris(dimethylamino)phosphonium-hexafluorophosphat.

Von den Verbindungen der allgemeinen Formel I, deren Aminogruppen geschützt sind, werden bevorzugt diejenigen, deren geschützte Aminogruppe Teil dieser Aminosäure $R^5$ sind.

Als Alkohol-Schutzgruppe kommen insbesondere substituierte oder unsubstituierte Methylether, Ethylether, Benzylether, Silylether, Ester, Carbonate oder Sulfonate in Frage.

Hierunter fallen folgende Verbindungen:

Als substituierte Methylether:

Methoxymethyl, Methylthiomethyl, t-Butylthiomethyl, (Phenyldimethylsilyl)methoxymethyl, Benzyloxymethyl, p-Methoxybenzyloxymethyl, (4-Methoxyphenoxy)-methyl, Guaiacolmethyl, t-Butoxymethyl, 4-Pentenyloxymethyl, Siloxymethyl, 2-Methoxyethoxymethyl, 2,2,2-Trichlorethoxymethyl, Bis(2-chlorethoxy)methyl, 2-(Trimethylsilyl)ethoxymethyl, Tetrahydropyranyl, 3-Bromotetrahydropyranyl, Tetrahydrothiopyranyl, 1-Methoxycyclohexyl, 4-Methoxytetrahydropyranyl, 4-Methoxytetrahydrothiopyranyl, 4-Methoxytetrahydrothiopyranyl-S,S-Dioxo, 1-[2-Chlor-4-methyl)-phenyl]-4-methoxypiperidin-4-yl, 1,4-Dioxan-2yl, Tetrahydrofuranyl, Tetrahydrothiofuranyl.

Als substituierte Ethylether:

1-Ethoxyethyl, 1-(2-Chlorethoxy)ethyl, 1-Methyl-1-methoxyethyl, 1-Methyl-1-benzyloxyethyl, 1-Methyl-1-benzyloxy-2-fluoroethyl, 2,2,2-Trichlorethyl, 2-Trimethylsilylethyl, 2-(Phenylselenyl)ethyl, t-Butyl, Allyl, p-Chlorphenyl, p-Methoxyphenyl, 2,4-Dinitrophenyl, Benzyl.

Als substituierte Benzylether:

p-Methoxybenzyl, 3,4-Dimethoxybenzyl, o-Nitrobenzyl, p-Nitrobenzyl, p-Halogenbenzyl, 2,6-Dichlorbenzyl, p-Cyanobenzyl, p-Phenylbenzyl, 2- und 4-Picolyl, 3-Methyl-2-picolyl-N-oxido, Diphenylmethyl, p,p'-Dinitrobenzhydryl, Triphenylmethyl, $\alpha$-Naphthyldiphenylmethyl, p-Methoxyphenyldiphenylmethyl, Di(p-methoxyphenyl)-phenylmethyl, Tri(p-methoxyphenyl)methyl, 4-(4'-Bromphenacyloxy)phenyldiphenylmethyl, 4,4',4''-Tris(4,5-dichlorphthalimidophenyl)methyl, 4,4',4''-Tris-(levulinooxyphenyl)methyl, 4,4',4''-Tris(benzoyloxyphenyl)methyl, 3-(Imidazol-1,4'-methyl)bis(4',4''-dimethoxyphenyl)-methyl, 1,1-Bis(4-methoxyphenyl)-1'-pyrenylmethyl, 9-Anthryl, 9-(9-Phenyl)-xanthenyl, 9-(9-Phenyl-10-oxo)anthryl.

Als Silylether:

Trimethylsilyl, Triethylsilyl, Triisopropylsilyl, Dimethylisopropylsilyl, Diethylisopropylsilyl, Dimethylthexylsilyl, t-Butyldimethylsilyl, t-Butyldiphenylsilyl, Tribenzylsilyl, Tri-p-xylylsilyl, Triphenylsilyl, Diphenylmethylsilyl, t-Butylmethoxyphenylsilyl.

Als Ester:

Formate, Benzoylformate, Acetate, Chloroacetat, Dichloroacetat, Trichloroacetat, Trifluoroacetat, Methoxyacetat, Triphenylmethoxyacetat, Phenoxyacetat, p-Chlorphenoxyacetat, p-P-Phenylacetat, 3-Phenylpropionat, 4-Oxopentanoat (Levulinat), 4,4-(Ethylendithio)pentanoat, Pivaloat, Adamantoat, Crotonat, 4-Methoxycrotonat, Benzoat, p-Phenylbenzoat, 2,4,6-Trimethylbenzoat (Mesitoat).

Als Carbonate:

Methyl, 9-Fluorenylmethyl, Ethyl, 2,2,2,-Trichlorethyl, 2-(Trimethylsilyl)ethyl, 2-(Phenylsulfonyl)ethyl, 2-(Triphenylphosphonio)ethyl, Isobutyl, Vinyl, Allyl, p-Nitrophenyl, Benzyl, p-Methoxybenzyl, 3,4-Dimethoxybenzyl, o-Nitrobenzyl, p-Nitrobenzyl, S-Benzyl Thiocarbonate, 4-Ethoxy-1-naphthyl, Methyl Dithiocarbonate.

Weitere Ester:

2,6-Dichlor-4-methylphenoxyacetat, 2,6-Dichlor-4-(1,1,3,3-tetramethylbutyl)phenoxyacetat, 2,4-Bis(1,1-dimethylpropyl)phenoxyacetat, Chlordiphenylacetat, Isobutyrat, Monosuccinat, (E)-2-Methyl-2-butenoat (Tigloat), O-(Methoxycarbonyl)benzoat, p-P-Benzoat, $\alpha$-Naphthoat, Nitrat, Alkyl N,N,N', N'-Tetramethylphosphorodiamidat, N-Phenylcarbamat, Borate, Dimethylphosphinothioyl, 2,4-Dinitrophenylsulfenat.

Als Sulfonate:

Sulfate, Methanesulfonat (Mesylat), Benzylsulfonat, Tosylate.

Insbesondere bevorzugt sind folgende Schutzgruppen:

$(C_1-C_6)$-Alkanoyl, $(C_1-C_8)$-Alkylcarbamoyl, Di-$(C_1-C_8)$-alkylcarbamoyl, N-$(C_3-C_8)$-cycloalkylcarbamoyl, $(C_1-C_6)$-Alkoxycarbonyl, $(C_6-C_{12})$-Aryloxycarbonyl, $(C_7-C_{11})$Aralkyloxycarbonyl, insbesondere Benzyloxycarbonyl, $(C_6-C_{12})$-Arylcarbonyl, $(C_7-C_{11})$-Aralkylcarbonyl, $(C_1-C_6)$-Alkyl, $(C_1-C_6)$-Alkoxy-$(C_1-C_6)$-alkyl, Carbamoyl-$(C_1-C_6)$-alkylester, $(C_1-C_{10})$-Acyloxy-$(C_1-C_6)$-alkyl, vorzugsweise $(C_1-C_{10})$-Alkanoyloxy-$(C_1-C_6)$-alkyl, Benzyloxy-$(C_1-C_6)$-alkyl, Benzyloxycarbonyloxy-$(C_1-C_6)$-alkyl $(C_1-C_6)$-Alkoxycarbonyloxy-$(C_1-C_6)$-alkyl, Aminosäureester oder Tetrahydropyranyl.

Bevorzugt sind Verbindungen der allgemeinen Formel I, in denen $R^1$ und/oder $R^3$ Wasserstoff oder Methyl und $R^2$ und/oder $R^4$ die oben angebenen Bedeutungen haben.

Bevorzugt sind weiterhin Verbindungen der allgemeinen Formel I, in denen $R^1$ und/oder $R^3$ Wasserstoff und $R^2$ und/oder $R^4$

A einen verzweigten oder unverzweigten ($C_1-C_{12}$)−Alkylrest bedeutet, der einfach oder mehrfach substituiert ist mit
($C_1-C_8$)−Alkoxycarbonyloxy, ($C_1-C_8$)−Alkoxy−($C_1-C_8$)−alkoxycarbonyloxy, ($C_6-C_{12}$)−Aryloxycarbonyloxy, ($C_7-C_{11}$)−Aralkyloxycarbonyloxy, ($C_7-C_{11}$)−Aralkylcarbonyloxy, ($C_7-C_{11}$)−Arylcarbonyloxy, ($C_3-C_8$)−Cycloalkylcarbonyloxy, ($C_1-C_{12}$)−Alkoxy−($C_1-C_{12}$)−alkoxy, Carbamoyloxy, N−($C_1-C_8$)−Alkylcarbamoyloxy, N,N−Di ($C_1-C_8$)−alkylcarbamoyl, N−($C_3-C_8$)−Cycloalkylcarbamoyl, N−($C_7-C_{11}$)−Aralkylcarbamoyloxy, N ($C_6-C_{12}$)−Arylcarbamoyloxy, wobei die in vorstehenden Substituenten vorhandenen Aryl− und Aralkylreste auch heterocyclischer Natur sein können und/oder wie auch Alkyl mit 1 oder 2 gleichen oder verschiedenen Substituenten aus der Reihe Halogen, Trifluormethyl, Hydroxy, ($C_1-C_3$)−Alkyl, ($C_1-C_3$)−Hydroryalkyl, ($C_1-C_9$)−Alkoxy, $-O-[CH_2-]_xC_fH_{(2f+1-g)}F_g$, $-OCF_2Cl$, $-O-CF_2-CHFCl$, −($C_1-C_3$)−Alkoxycarbonyl, Carbamoyl, ($C_1-C_6$)−Alkylcarbonyloxy, ($C_3-C_8$)−Cycloalkyl, Phenyl, Benzyl, Phenoxy, Benzyloxy, substituiert sind, oder mit
einem substituierten ($C_6-C_{12}$)−Arylrest oder Heteroarylrest, der 1 oder 2 gleiche oder verschiedene Substituenten aus der Reihe Hydroxy, Trifluormethyl, ($C_1-C_3$)−Hydroxyalkyl, ($C_1-C_3$)−Alkoxycarbonyl, Carbamoyl, NR'R'', N−($C_1-C_4$)−Alkylcarbamoyl, N,N−Di−($C_1-C_4$)−alkylcarbamoyl, ($C_1-C_3$)−Alkylcarbonyloxy, Aminoalkyl oder N−($C_1-C_4$)−alkylamino−($C_1-C_6$)−alkyl trägt, wobei R' und R'' gleich oder verschieden sind und Wasserstoff, ($C_6-C_{12}$)−Aryl, oder ($C_1-C_4$)−Alkyl bedeuten, oder mit
einem substituierten ($C_6-C_{10}$)−Aryloxyrest oder ($C_7-C_{11}$)−Aralkyloxyrest, der 1 oder 2 gleiche oder verschiedene Substituenten aus der Reihe Hydroxy, Halogen, Trifluormethyl, ($C_1-C_3$)−Alkyl, ($C_1-C_3$)−Hydroxyalkyl, ($C_1-C_3$)−Alkoxy, ($C_1-C_3$)−Alkylmercapto, ($C_1-C_3$)−Alkylsulfinyl, ($C_1-C_3$)−Alkylsulfonyl, ($C_1-C_3$)−Alkylcarbonyl, ($C_1-C_3$)−Alkoxycarbonyl, Carbamoyl, N−($C_1-C_4$)−Alkylcarbamoyl, N,N−Di−($C_1-C_4$)−alkylcarbamoyl, ($C_1-C_3$)−Alkylcarbonyloxy, NR'R'' trägt, wobei R' und R'' gleich oder verschieden sind und Wasserstoff, ($C_6-C_{10}$)−Aryl oder ($C_1-C_4$)−Alkyl bedeuten, oder mit
einem Rest der allgemeinen Formel II

$$-O-R^5 \quad (II)$$

worin
$R^5$ eine über ihren Acylrest gebundene Aminosäure oder ein Derivat hiervon bedeutet,
B einen ($C_6-C_{12}$)Aryl− oder ($C_7-C_{11}$)Aralkylrest, vorzugsweise Phenyl, Benzyl und Phenethyl, bedeuten, welche einfach substituiert sind mit Hydroxy, ($C_1-C_4$)−Alkylcarbonyloxy, ($C_1-C_4$)−Alkoxycarbonyl, ($C_1-C_4$)−Hydroxyalkyl, Amino, ($C_1-C_5$)−Alkylamino, Di−($C_1-C_5$)alkylamino, ($C_1-C_5$)−Alkanoylamino, Carbamoyl, N−($C_1-C_4$)−Alkylcarbamoyl, N,N−Di−($C_1-C_4$)−alkylcarbamoyl, Carbamoyl, N−($C_1-C_4$)−Alkylcarbamoyloxy, N,N−Di−($C_1-C_4$)−alkylcarbamoyloxy, oder
C einen ($C_1-C_6$)Alkoxyrest, ($C_3-C_8$)−Cycloalkoryrest, ($C_6-C_{12}$)−Aryloxyrest und ($C_7-C_{11}$)−Aralkyloxyrest bedeuten,

- n = 0 oder 1,
- f = 1 bis 8, vorzugsweise 1 bis 5,
- g = 0, 1 bis (2f + 1),
- x 0, 1, 2 oder 3, vorzugsweise 0 oder 1 ist und

wobei die genannten Reste $R^1$, $R^2$, $R^3$ und $R^4$ in Kombination mit einem
($C_1-C_{12}$)−Alkylrest, der einfach oder mehrfach, vorzugsweise ein− oder zweifach, mit Wasserstoff, Halogen, Hydroxy, Amino, ($C_1-C_4$)−Alkoxy, ($C_1-C_4$) Alkoxycarbonyl, ($C_1-C_4$) Alkyl− oder ($C_1-C_4$) Dialkylamino oder einem Phenylring, der ein−, zwei− oder dreifach mit den Resten Halogen, Nitro, ($C_1-C_4$)−Alkyl oder ($C_1-C_4$)−Alkoxy substituiert ist, auftreten können und auch in Kombination mit einem Aryl− oder Heteroarylrest, der seinerseits gegebenenfalls mit Halogen, ($C_1-C_4$)−Alkyl oder ($C_1-C_4$)−Alkoxy 1− oder 2−fach substituiert sein kann, zuzüglich alle Derivate, die in der entsprechenden Amino− oder Hydroxygruppe eine Schutzgruppe aufweisen, sowie die physiologisch wirksamen Salze.

Insbesondere bevorzugt sind Verbindungen der allgemeinen Formel I, in denen $R^1$ und/oder $R^3$ Wasserstoff und $R^2$ und/oder $R^4$
A einen unverzweigten ($C_1-C_{12}$)−Alkylrest bedeutet, der einfach substituiert ist mit
($C_1-C_8$)−Alkoxycarbonyloxy, ($C_1-C_8$)−Alkoxy−($C_1-C_8$)−alkoxycarbonyloxy, ($C_6-C_{12}$)−Aryloxycarbonyloxy, ($C_7-C_{11}$)−Aralkyloxycarbonyloxy, ($C_7-C_{11}$)−Aralkylcarbonyloxy, ($C_6-C_{12}$)−Arylcarbonyloxy, ($C_3-C_8$)−Cycloalkylcarbonyloxy, ($C_1-C_{12}$)−Alkoxy−($C_1-C_{12}$)−alkoxy, ($C_1-C_{12}$)−Alkoxy−amino, ($C_1-C_{12}$)−Alkoxy−N−($C_1-C_6$)−alkylamino, ($C_1-C_{12}$)−Alkoxy−N,N−($C_1-C_6$)−dialkylamino, N,N−Di−($C_1-C_8$)−alkylcarbamoyl, N−($C_3-C_8$)−Cycloalkylcarbamoyl, N($C_7-C_{11}$)−Aralkylcarbonyloxy, N−($C_6-C_{12}$)−Arylcarbamoyloxy, ($C_1-C_5$)−Alkanoylamino, ($C_3-C_6$)−Cycloalkanoylamino, ($C_6-C_{12}$)−Aroylamino, ($C_7-C_{11}$)−Aralkanoylamino, wobei Alkyl, Aryl, Aryloxy, Aralkyl oder Aralkyloxy, ihrerseits substituiert sind

mit Hydroxy, Halogen, insbesondere Fluor, $(C_1-C_3)$Alkyl, $(C_1-C_3)$Alkoxy, oder mit
einem einfach mit einer Hydroxy-Gruppe substituierten Phenylrest, einem substituierten Phenoxy- oder Benzyloxyrest, substituiert mit Hydroxy, Halogen, $(C_1-C_4)$ Alkoxy, oder mit
einem Rest der allgemeinen Formel II

$-O-R^5$ (II)

worin $R^5$ eine über ihren Acylrest gebundene Aminosäure oder ihr an der Aminogruppe substituiertes Derivat bedeutet,
B einen $(C_6-C_{12})$-Aryl- oder $(C_7-C_{11})$-Aralkylrest, vorzugsweise Phenyl, Benzyl und Phenethyl, bedeuten, welcher einfach substituiert ist mit Hydroxy und
C Methoxy bedeutet.

Weiterhin betrifft die Erfindung ein Verfahren zur Herstellung von Verbindungen der Formel I, das dadurch gekennzeichnet ist, daß man
eine Verbindung der Formel IV

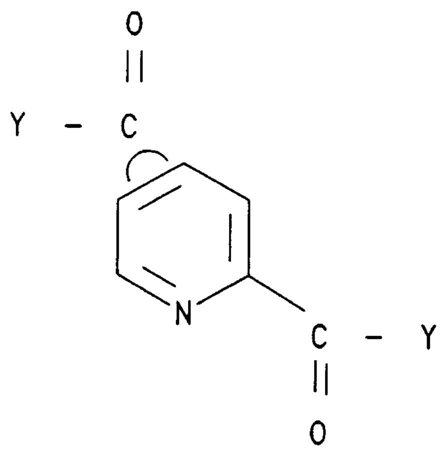

(IV)

mit einer Verbindung der Formeln V

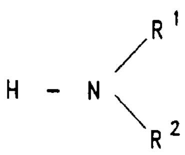

oder

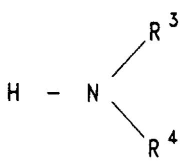

(V)

umsetzt, wobei $R^1$, $R^2$ bzw. $R^3$, $R^4$ die zu Formel I angegebenen Bedeutungen haben und Y Halogen oder Hydroxy ist oder zusammen mit der Carbonylgruppe einen Aktivester oder ein gemischtes Anhydrid bildet, und die Reaktionsprodukte gegebenenfalls in ihre physiologisch verträglichen Salze überführt.

Im Folgenden wird die Herstellung von Verbindungen gemäß Formel I und die Herstellung der dafür benötigten Ausgangssubstanzen - sofern sie nicht käuflich sind - näher beschrieben.

Die Herstellung der erfindungsgemäßen Verbindungen gelingt am einfachsten dadurch, daß die beiden Komponenten, das Pyridin-Derivat gemäß Formel (IV) und das Amin gemäß Formel (V) in äquimolaren Mengen oder bis zu einem etwa 5-fachen Überschuß an V, vermengt werden und bei Temperaturen zwischen -30 und 150°C, bevorzugt bei 20 bis 100°C bis zur Beendigung der Reaktion umgesetzt werden. Die Beendigung der Reaktion läßt sich mittels Dünnschichtchromatographie (DC-Kontrolle) bestimmen. Eine Variante dieses Verfahrens besteht darin, daß man in einem gee gneten Lösungsmittel, wie Diethylether oder Dimethoxyethan oder Tetrahydrofuran, chlorierten Kohlenwasserstoffen wie Methylenchlorid, Chloroform, Tri- oder Tetrachlorethylen, Benzol, Toluol oder auch polaren Lösungsmitteln wie Dimethylformamid oder Aceton oder Dimethylsulfoxid arbeitet.

Auch hier kann ein Überschuß von Amin gemäß Formel (V), der bis zur etwa 5-fachen Mengen betragen kann, angewandt werden. Die Reaktionstemperaturen liegen dabei zwischen Raumtemperatur und dem Siedepunkt des Lösungsmittels, wobei Temperaturen im Bereich von Raumtemperatur bis 130 °C besonders bevorzugt sind.

Ebenso kann die Umsetzung über ein gemischtes Anhydrid wie Chlorameisensäureethylester oder über einen aktivierten Ester wie Paranitrophenylester (Y = $ClCH_2-COO$ oder $NO_2-C_6H_4-O$) erfolgen. Entsprechende Methoden sind in Houben-Weyl, Methoden der Organischen Chemie, Band XV/2, Seiten 169 bis 183 (gemischte Anhydridmethode) bzw. Seiten 13 ff. (Aktivestermethode), vierte Auflage, Georg Thieme Verlag, Stuttgart 1974, beschrieben.

Gegebenenfalls kann die Umsetzung auch in Gegenwart von Basen erfolgen. Als zusätzliche Basen kommen anorganische Säurefänger wie Carbonate oder Hydrogencarbonate z B. Natrium- oder Kalium-carbonat oder Natrium- oder Kaliumhydrogencarbonat, oder organische Säurefänger wie tertiäre Amine, wie Triethylamin, Tributylamin, Ethyldiisopropylamin oder heterocyclische Amine wie N-Alkylmorpholin, Pyridin, Chinolin oder Dialkylaniline in Betracht.

Bevorzugt erfolgt die Umsetzung der Verbindungen gemäß Formel (IV) mit Aminen gemäß Formel (V) unter Zusatz eines wasserabspaltenden Mittels wie Dialkylcarbodiimid, wobei die Alkylreste 1 bis 8 C-Atome aufweisen, die im Falle der $C_3-C_8$-Verbindungen auch verzweigt oder cyclisch sein können; bevorzugt wird Dicyclohexylcarbodiimid eingesetzt. Eine entsprechende Methode ist in Houben-Weyl Bd. XV/2, Seiten 103 bis 111, Methoden der Organischen Chemie, 4. Auflage, Georg Thieme Verlag, Stuttgart, 1974, beschrieben.

Gegebenenfalls kann die Aufarbeitung der Produkte beispielsweise durch Extraktion oder durch Chromatographie z. B. über Kieselgel erfolgen. Das isolierte Produkt kann umkristallisiert und gegebenenfalls mit einer geeigneten Säure zu einem physiologisch verträglichen Salz umgesetzt werden. Als geeignete Säuren kommen beispielsweise in Betracht:

Mineralsäuren, wie Chlorwasserstoff- und Bromwasserstoffsäure sowie Schwefel-, Phosphor-, Salpeter- oder Perchlorsäure oder organische Säuren wie Ameisen-, Essig-, Propion-, Bernstein-, Glykol-, Milch-, Äpfel-, Wein-, Zitronen-, Malein-, Fumar-, Phenylessig-, Benzoe-, Methansulfon-, Toluolsulfon-, Oxal-, 4-Aminobenzoe-, Naphthalin-1,5-disulfon- oder Ascorbinsäure.

Die Ausgangsverbindungen der Formel (V) können, soweit sie nicht käuflich sind, einfach synthetisiert werden (z. B. Organikum, Organisch Chemisches Grundpraktikum, 15. Auflage, VEB Deutscher Verlag der Wissenschaften, 1976; eine Übersicht über die verschiedenen Möglichkeiten findet sich im Methodenregister, Seite 822).

Die Ausgangsverbindung der Formel (IV) erhält man beispielsweise durch Umsetzung von Pyridin-2,4- oder -2,5-dicarbonsäure zu dem entsprechenden Pyridin-2,4- oder -2,5 dicarbonsäurehalogenid, bevorzugt -chlorid (nach literaturbekannten Verfahren, z. B. Organikum, Organisch Chemisches Grundpraktikum, 15. Auflage, VEB Deutscher Verlag der Wissenschaften, 1976, Seite 595 ff), welches dann mit einem geeigneten Alkohol, z. B. Paranitrobenzylalkohol zu dem entsprechenden Aktivester umgesetzt wird. Ebenso kann die Pyridin-2,4- oder -2,5-dicarbonsäure auch zunächst unter Zusatz einer geeigneten Carbonsäure oder Carbonsäureester wie Chlorameisensäureethylester in ein gemischtes Anhydrid überführt werden, welches dann mit den Aminen (V) zu den erfindinngsgemäßen Produkten umgesetzt wird. Eine entsprechende Methode ist z. B. in Houben-Weyl, Methoden der organischen Chemie, Bd. XV/2, Seiten 169 bis 183, 4. Auflage, 1974, Georg Thieme Verlag Stuttgart, beschrieben.

Die erfindungsgemäßen Verbindungen der Formel I besitzen wertvolle pharmakologische Eigenschaften und zeigen insbesondere Wirksamkeit als Hemmer der Prolin- und Lysinhydroxylase, als Fibrosuppressivum, Immunsuppressivum, und Antiatherosklerotikum.

Die antifibrotische Wirkung kann im Modell der Tetrachlorkohlenstoff-induzierten Leberfibrose bestimmt werden. Dazu werden Ratten mit $CCl_4$ (1 ml/kg) - gelöst in Olivenöl - zweimal wöchentlich behandelt. Die Prüfsubstanz wird täglich, gegebenenfalls sogar zweimal täglich per os oder intraperitoneal - gelöst in einem geeigneten verträglichen Lösungsmittel - verabreicht. Das Ausmaß der Leberfibrose wird histologisch bestimmt und der Anteil Kollagen in der Leber per Hydroxyprolinbestimmung - wie bei Kivirikko et al. (Anal. Biochem. 19, 249 f. (1967)) beschrieben - analysiert. Die Aktivität der Fibrogenese kann durch radioimmunologische Bestimmung von Kollagenfragmenten und Prokollagenpeptiden im Serum bestimmt werden. Die erfindungsgemäßen Verbindungen sind in diesem Modell in Konzentration 1 - 100 mg/kg wirksam.

Die Aktivität der Fibrogenese kann durch radioimmunologische Bestimmung des N-terminalen Propeptids des Kollagens Typ-III oder der N- bzw. C-terminalen Quervernetzungsdomäne des Kollagens-Typ-IV (7s-Kollagen bzw. Typ-IV-Kollagen-$NC_1$) im Serum bestimmt werden.

Zu diesem Zweck wurden die Hydroxyprolin-, Prokollagen-III-Peptid-, 7s-Kollagen- und Typ-IV-Kollagen-NC-Konzentrationen in der Leber von

a) unbehandelten Ratten (Kontrolle)

b) Ratten, denen Tetrachlorkohlenstoff verabreicht wurde ($CCl_4$-Kontrolle)

c) Ratten, denen zunächst $CCl_4$ und anschließend eine erfindungsgemäße Verbindung verabreicht wurde

gemessen (diese Testmethode wird beschrieben von Rouiller, C., experimental toxic injury of the liver; in The Liver, C. Rouiller, Vol. 2, 5. 335 - 476, New York, Academic Press, 1964).

Ein anderes Modell zur Evaluierung der antibiotischen Wirkung ist das der Bleomycin-induzierten Lungenfibrose wie bei Kelley et al. (J. Lab. Clin. Med. 96, 954, (1980)) beschrieben. Für die Evaluierung der Wirkung der erfindungsgemäßen Verbindungen der Granulationsgewebe kann das Modell des Wattebauschgranuloms, wie bei Meier et al., Experimentia 6, 469 (1950) beschrieben, herangezogen werden.

Die Verbindungen der Formel I können als Medikamente in Form pharmazeutischer Präparate Verwendung finden, welche sie gegebenenfalls zusammen mit verträglichen pharmazeutischen Trägern enthalten. Die Verbindungen können als Heilmittel, z.B. in Form pharmazeutischer Präparate Verwendung finden, welche diese Verbindungen in Mischung mit einem für die enterale, perkutane oder parenterale Applikation geeigneten pharmazeutischen, organischen oder anorganischen Träger, wie z.B. Wasser, Gummi arabicum, Gelatine, Milchzucker, Stärke, Magnesiumstearat, Talk, pflanzliche Öle, Polyalkylenglykole, Vaseline usw. enthalten.

Sie können zu diesem Zweck oral in Dosen von 0,1 - 25 mg/kg/Tag, vorzugsweise 1 - 5 mg/kg/Tag oder parenteral in Dosen von 0,01 - 5 mg/kg/Tag, vorzugsweise 0,01 - 2,5 mg/kg/Tag inbesondere 0,5 - 1,0 mg/kg/Tag, appliziert werden. Die Dosierung kann in schweren Fällen auch erhöht werden. In vielen Fällen genügen jedoch auch geringere Dosen. Diese Angaben beziehen sich auf einen Erwachsenen von etwa 75 kg Gewicht.

Die Erfindung umfaßt weiterhin die Verwendung der erfindungsgemäßen Verbindungen bei der Herstellung von Arzneimitteln, die zur Behandlung und Prophylaxe der vorstehend genannten Stoffwechselstörungen eingesetzt werden.

Ein weiterer Gegenstand der Erfindung sind Arzneimittel, die ein oder mehrere erfindungsgemäße Verbindungen der Formel I und/oder deren physiologisch verträgliche Salze enthalten.

Die Arzneimittel werden nach an sich bekannten, dem Fachmann geläufigen Verfahren hergestellt. Als Arzneimittel werden die erfindungsgemäßen pharmakologisch wirksamen Verbindungen (= Wirkstoff) entweder als solche oder vorzugsweise in Kombination mit geeigneten pharmazeutischen Hilfs- oder Trägerstoffen in Form von Tabletten, Dragees, Kapseln, Suppositorien, Emulsionen, Suspensionen oder Lösungen eingesetzt, wobei der Wirkstoffgehalt bis etwa 95 %, vorteilhafterweise zwischen 10 und 75 % beträgt.

Geeignete Hilfs- bzw. Trägerstoffe für die gewünschte Arzneimittelformulierung sind beispielsweise neben Lösemitteln, Gelbildnern, Suppositoriengrundlagen, Tabletten-Hilfsstoffen und anderen Wirkstoffträgern auch Antioxidantien, Dispergiermittel, Emulgatoren, Entschäumer, Geschmackskorrigentien, Konservierungsmittel, Lösungsvermittler oder Farbstoffe.

Im folgenden wird die Erfindung anhand von Beispielen näher erläutert.

Beispiel 1

Pyridin-2,4-dicarbonsäure-bis-N,N'-(3-benzoyloxypropyl)amid

a) 25 g (128 mMol) Pyridin-2,4-dicarbonsäuredimethylester werden in 500 ml Ethanol gelöst und mit 22 ml (282 mMol) 3-Amino-1-propanol 4 Stunden zum Rückfluß erhitzt.

Nachdem über Nacht bei Raumtemperatur stehen gelassen wurde, destilliert man das Lösungsmittel in Vak. ab und kristallisiert den Rückstand heiß aus Ethylacetat; 28,7 g Fp. 102 ° - 105 °C.

b) 0,7 g (2,5 mM) des so erhaltenen Pyiridin-2,4-dicarbonsäure-bis-N,N'-(3-hydroxy-propyl)-amids werden in 100 ml Dichlormethan mit 0,2 g 4-N,N-Dimethylaminopyridin, 0,8 ml (6 mM) Triethylamin und sodann tropfenweise mit 0,6 ml (5 mM) Benzoylchlorid versetzt. Nach 1 Stunde wird eingeengt mit Wasser aufgenommer.

Nach 1 Stunde wird zweimal mit Wasser ausgeschüttelt und die organische Phase eingeengt. Das Rohprodukt wird mit Ethylacetat an Kieselgel chromatographiert, Ausbeute: 0,95 g farbloses Öl.

Summenformel: $C_{27} H_{27} N_3 O_6$ (489)

MS: m/e = 490 (M + $H^+$)

Beispiel 2

Pyridin – 2,4 – dicarbonsäure – bis – N,N' – [2 – (2 – methylbenzoyloxy)propyl]amid

Man erhält die Titelverbindung analog Beispiel 1 aus 0,7 g (2,5 mM) Pyridin – 2,4 – dicarbonsäure – bis – N,N' – (3 – hydroxypropyl)amid und 0,66 ml (5 mM) 2 – Methylbenzoylchlorid, Ausbeute: 0,90 g farbloses Öl.
Summenformel: $C_{29}$ $H_{31}$ $N_3$ $O_6$ (517)
MS. m/e = 518 (M + $H^+$)

Beispiel 3

Pyridin – 2 – carbonsäure – (3 – methoxypropyl)amid – 4 – carbonsäure – [3 – (2 – methylbenzoyloxy)propyl] – amid

10,3 g (40 mM) Pyridin – 2 – carbonsäure – 4 – carbonsäurebenzylester werden in 160 ml wasserfreiem Tetrahydrofuran gelöst und bei 0 °C mit 6 ml (43 mM) Triethylamin versetzt. Nach 10 min werden 4,1 ml (43 mM) Chlorameisensäureethylester zugetropft und 30 min bei 0 °C gerührt.
Dann gibt man 4,4 ml (43 mM) 3 – Methoxypropylamin zu, rührt 1 Stunde bei 0 °C, engt im Vakuum bei Raumtemperatur ein, nimmt in Dichlormethan auf, wäscht mit gesättigter $NaHCO_3$ – Lösung, trocknet, befreit vom Lösungsmittel und erhält 11,2 g Pyridin – 2 – carbonsäure – (3 – methoxypropyl)amid – 4 – carbonsäurebenzylester. 6,0 g (18,3 mM) dieser Verbindung werden in 15 ml 3 – Amino – 1 – propanol 1 Stunde bei 80 °C gerührt. Das überschüssige Reagenz wird im Vakuum abdestilliert und der Rückstand aus Ethylacetat kristallisiert, Ausbeute: 4,8 g Pyridin – 2 – carbonsäure – (3 – methoxypropyl)amid – 4 – carbonsäure – (3 – hydroxypropyl)amid,
Fp. 71 – 73 °C
2,0 g dieser Verbindung werden analog Beispiel 1 mit 2 – Methylbenzoylchlorid acyliert. Man erhält 2,4 g der Titelverbindung als farbloses, öliges Produkt, nach Chromatographie mit Ethylacetat an Kieselgel.
Summenformel: $C_{22}$ $H_{27}$ $N_3$ $O_5$ (413,5)
MS: m/e = 414 (M + $H^+$)

Beispiel 4

Pyridin – 2 – carbonsäure – (3 – hydroxypropyl)amid – 4 – carbonsäure – [3 – (2 – methylbenzoyloxy)propyl] – amid

1,6 g (3,86 mM) der Titelverbindung aus Beispiel 3 werdenin 50 ml Dichlormethan bei – 25 °C tropfenweise mit 4,5 ml 1 M Bortribromid – Lösung in Hexan (4,5 mM) versetzt. Nach DC – Kontrolle werden weitere 1,5 ml dieser Lösung zugetropft, nach 0,5 Stunden auf Raumtemperatur erwärmt, mit gesättigter $NaHCO_3$ – Lösung ausgeschüttelt, die organische Phase getrocknet, eingeengt und der ölige Rückstand mit Ethylacetat/Methanol an Kieselgel chromatographiert. 0,61 g der Titelverbindung kristallisieren aus Ethylacetat,
Fp. 78 – 80 °C
Summenformel: $C_{21}$ $H_{25}$ $N_3$ $O_5$ (399,4)
MS: m/e = 400 (M + $H^+$)

Beispiel 5

Pyridin – 2 – carbonsäure – (3 – methoxypropyl)amid – 4 – carbonsäure – 3 – (N – cyclohexylcarbamoyloxy) – propyl – amid

2,0 g (6,8 mM) Pyridir – 2 – carbonsäure – (3 – methoxypropyl)amid – 4 – carbonsäure – (3 – hydroxypropylamid (vgl. Beispiel 3) werden in 250 ml Dichlormethan gelöst und unter Rühren bei 0 °C mit 1,05 ml (7,5 mM) Cyclohexylisocyanat versetzt. Anschließend erhitzt man 1 Stunde zum Rückfluß, kontrolliert den Umsatz mittels DC, versetzt mit weiteren 1,1 ml Cyclohexylisocyanat, erwärmt eine weitere Stunde, läßt abkühlen, versetzt mit Wasser, trocknet die organische Phase, engt ein und chromatographiert der öligen Rückstand mit Ethylacetat an Kieselgel. Entsprechende Fraktionen werden eingeengt und mit Diethylether zur Kristallisation gebracht; Ausbeute: 1,1 g der farblos kristallinen Titelverbindung, Fp. 95 – 98 °C.

Beispiel 6

Pyridin – 2 – carbonsäure – (2 – methoxyethyl) – amid – 4 – carbonsäure – (3 – benzoyloxyethyl) – amid

a) 150 g Pyiridin – 2,4 – dicarbonsäure werden in 1,8 l Methanol und 53,5 g Schwefelsäure (98 %) 3 Stunden zum Sieden erhitzt. Nach 1 h ist weitgehend die Lösung erfolgt. Das Gemisch ist nur noch leicht trübe. Man gießt in Eiswasser, läßt den feinkristallinen Niederschlag absetzen, dekantiert, saugt dann ab, wäscht mit Wasser und danach mit sehr wenig eiskaltem MeOH. Ausbeute 70 – 75 g Pyridin – 2 – carbonsäuremethylester – 4 – carbonsäure, Fp. 246 – 248 ° C (Zers.). Das Filtrat wird 3 x mit $CH_2Cl_2$ extrahiert die org. Phasen getrocknet, eingedampft, in Ethylacetat aufgenommen und über Kieselgel (ca. 1 kg) filtriert. Das Filtrat wird im Vakuum eingedampft. Ausbeute 90 – 95 g Pyridin – 2,4 – dicarbonsäure – dimethylester, Fp. 61 – 62 ° C.

b) Aus 50 g Pyridin – 2 – carbonsäuremethylester – 4 – carbonsäure und 150 ml 2 – Methoxyethylamin erhält man 32,5 g Pyridin – 2 – carbonsäure – (2 – methoxyethyl)amid – 4 – carbonsäure, Fp. 145,5 – 146 ° C (aus Wasser).

c) 30 g der vorstehenden Verbindung werden mit 10 ml Thionylchlorid in 180 ml Toluol und 1 Tropfen Dimethylformamid 3 Stunden erhitzt. Nach Abkühlen gibt man 39 ml Triethylamin, sodann wasserfreies Methanol zu. Nach Filtration mit Ethylacetat über Kieselgel und Umkristallisieren aus Methanol/Wasser erhält man 19,8 g Pyridin – 2 – carbonsäure – (2 – methoxyethyl)amid – 4 – carbonsäuremethylester, Fp. 68 – 68,5 ° C.

d) 5,0 g der vorstehenden Verbindung werden in 10 ml Ethanolamin 30 min zum Sieden erhitzt. Man erhält 3,0 g Pyridin – 2 – carbonsäure – (2 – methoxyethyl)amid – 4 – carbonsäure – (2 – hydroxyethyl)amid, Fp. 124 – 125 ° C, in Form farbloser Kristalle.

e) Aus vorstehender Verbindung erhält man die Titelverbindung analog Beispiel 1 durch Reaktion mit Benzoylchlorid in Gegenwart von Triethylamin und 4 – N,N – Dimethylaminopyridin als farblose Kristalle, Fp. 77 – 78 ° C
Summenformel $C_{19}H_{21}N_3O_5$ (371)
MS: m/e = 372 (M + $H^+$).

Beispiel 7

Pyridin – 2 – carbonsäure – (2 – methoxyethyl)amid – 4 – carbonsäure – (2 – benzoyloxy – propyl) amid

Die Verbindung erhält man analog Beispiel 6 d) und 6 e) als farbloses Öl,
Summenformel $C_{20}H_{23}N_3O_5$ (385);
MS: m/e = 386 (M + $H^+$).

Beispiel 8

Pyridin – 2 – carbonsäure – (2 – methoxyethyl) – amid – 4 – carbonsäure – [2 – (4 – hydroxyphenyl) – ethyl] – amid

Die Titelverbindung erhält man aus 0,24 g Pyridin – 2 – carbonsäure – (2 – methoxyethyl) – amid – 4 – carbonsäuremethylester (vgl. Beispiel 6 c)) durch Schmelzen mit 2 – (4 – Hydroxyphenyl)ethylamin (Tyramin). Nach Filtrieren mit Ethylacetat über Kieselgel erhält man 70 mg farblose Nadeln; Fp. 181 – 181,5 ° C (aus Ethylacetat);
Summenformel $C_{18}H_{21}N_3O_4$ (343)
MS: m/e = 344 (M + $H^+$).

Beispiel 9

Pyridin – 2,4 – dicarbonsäure – bis – N,N' – [2 – (4 – methylbenzoyloxy) – ethyl] – amid

Die Titelverbindung erhält man analog Beispiel 1 b) aus Pyridin – 2,4 – dicarbonsäure – bis (N,N' – (2 – hydroxyethyl)amid und 4 – Methylbenzoylchlorid als farbloses Kristallpulver, Fp. 165 – 166 ° C
Summenformel: $C_{27}H_{27}N_3O_6$ (489)
MS: m/e = 490 (M + $H^+$).

Beispiel 10

Pyridin – 2,4 – dicarbonsäure – bis – N,N' – (2 – benzoyloxyethyl) – amid

analog Beispiel 1 b)
farblose Nadeln, Fp` 139 – 140 ° C
Summenformel: $C_{25}H_{23}N_3O_6$ (461)
MS: m/e = 462 (M + $H^+$).

In den folgenden Tabellen steht für

| | |
|---|---|
| Ph | Phenyl |
| Me | Methyl |
| Et | Ethyl |
| Pr | Propyl |
| Bu | Butyl |
| Bn | Benzyl |
| Pen | Pentyl |
| Hex | Hexyl |
| THP | Tetrahydropyranoyl |
| n | unverzweigte Kette |
| c | Cyclo |
| i | iso. |

$R^1/R^2$ bzw. $R^3/R^4$ bedeutet, daß entweder $R^1$ oder $R^2$ bzw. $R^3$ oder $R^4$ den genannten Rest aufweist. Der jeweils verbleibende Substituent ist Wasserstoff.

Es handelt sich jeweils um 2,4 – disubstituierte Pyridinderivate.

| Bspl. | $R^1/R^2$ | $R^3/R^4$ |
|---|---|---|
| 11 | $CH_2$ $CH_2$ O CO Ph | $CH_2$ $CH_2$ O CO Ph |
| 12 | $CH_2$ $CH_2$ O CO Ph | $CH_2$ $CH_2$ O CO NH Et |
| 13 | $CH_2$ $CH_2$ O CO Ph | $CH_2$ $CH_2$ O CO NH-nBu |
| 14 | $CH_2$ $CH_2$ O CO OEt | $CH_2$ $CH_2$ O CO Ph |
| 15 | $CH_2$ $CH_2$ O CO OEt | $CH_2$ $CH_2$ O CO NH c-Hex |
| 16 | $CH_2$ $CH_2$ O CO NH n-Bu | $CH_2$ $CH_2$ O CO Ph |
| 17 | $CH_2$ $CH_2$ O CO NH n-Bu | $CH_2$ $CH_2$ O CO NH n-Bu |
| 18 | $CH_2$ $CH_2$ O CO NH $CH_2$ $CH_2$ OH | $CH_2$ $CH_2$ O CO NH n-Bu |
| 19 | $CH_2$ $CH_2$ O CO NH $CH_2$ $CH_2$ OH | $CH_2$ $CH_2$ O CO NH $CH_2$ $CH_2$ OH |
| 20 | $CH_2$ $CH_2$ O THP | $CH_2$ $CH_2$ O THP |
| 21 | $CH_2$ $CH_2$ O THP | $CH_2$ $CH_2$ O CO Ph |
| 22 | $CH_2$ $CH_2$ $CH_2$ O CO Ph | $CH_2$ $CH_2$ $CH_2$ O CO Ph |
| 23 | $CH_2$ $CH_2$ $CH_2$ O CO Ph | $CH_2$ $CH_2$ $CH_2$ O CO NH Et |
| 24 | $CH_2$ $CH_2$ $CH_2$ O CO Ph | $CH_2$ $CH_2$ $CH_2$ O CO NH n-Bu |
| 25 | $CH_2$ $CH_2$ $CH_2$ O CO O Et | $CH_2$ $CH_2$ $CH_2$ O CO Ph |
| 26 | $CH_2$ $CH_2$ $CH_2$ O CO O Et | $CH_2$ $CH_2$ $CH_2$ O CO NH c-Hex |
| 27 | $CH_2$ $CH_2$ $CH_2$ O CO NH n-Bu | $CH_2$ $CH_2$ $CH_2$ O CO Ph |
| 28 | $CH_2$ $CH_2$ $CH_2$ O CO NH n-Bu | $CH_2$ $CH_2$ $CH_2$ O CO NH n-Bu |

| Bspl. | $R^1/R^2$ | $R^3/R^4$ |
|---|---|---|
| 29 | $CH_2$ $CH_2$ $CH_2$ O CO NH $CH_2$- $CH_2$ OH | $CH_2$ $CH_2$ $CH_2$ O CO NH n-Bu |
| 30 | $CH_2$ $CH_2$ $CH_2$ O CO NH $CH_2$- $CH_2$ OH | $CH_2$ $CH_2$ $CH_2$ O CO NH $CH_2$ $CH_2$ OH |
| 31 | $CH_2$ $CH_2$ $CH_2$ O THP | $CH_2$ $CH_2$ $CH_2$ O THP |
| 32 | $CH_2$ $CH_2$ $CH_2$ O THP | $CH_2$ $CH_2$ $CH_2$ O CO Ph |
| 33 | $CH_2$ $CH_2$ O $CH_3$ | $CH_2$ $CH_2$ $CH_2$ O CO c-Hex |
| 34 | $CH_2$ $CH_2$ O $CH_3$ | $CH_2$ $CH_2$ $CH_2$ O CO c-Pen |
| 35 | $CH_2$ $CH_2$ O $CH_3$ | $CH_2$ $CH_2$ $CH_2$ O CO O Et |
| 36 | $CH_2$ $CH_2$ O $CH_3$ | $CH_2$ $CH_2$ $CH_2$ O CO O n-Bu |
| 37 | $CH_2$ $CH_2$ O $CH_3$ | $CH_2$ $CH_2$ $CH_2$ O CO O c-Hex |
| 38 | $CH_2$ $CH_2$ O $CH_3$ | $CH_2$ $CH_2$ $CH_2$ O CO Ph |
| 39 | $CH_2$ $CH_2$ O $CH_3$ | $CH_2$ $CH_2$ $CH_2$ O CO i-Pr |
| 40 | $CH_2$ $CH_2$ O $CH_3$ | $CH_2$ $CH_2$ $CH_2$ O CO O Ph |
| 41 | $CH_2$ $CH_2$ O $CH_3$ | $CH_2$ $CH_2$ $CH_2$ O $CH_2$ $CH_2$ O Me |
| 42 | $CH_2$ $CH_2$ O $CH_3$ | $CH_2$ $CH_2$ $CH_2$ O $CH_2$ $CH_2$ O Et |
| 43 | $CH_2$ $CH_2$ O $CH_3$ | $CH_2$ $CH_2$ $CH_2$ O $CH_2$ $CH_2$ O Ph |
| 44 | $CH_2$ $CH_2$ O $CH_3$ | $CH_2$ $CH_2$ $CH_2$ O Ph |
| 45 | $CH_2$ $CH_2$ O $CH_3$ | $CH_2$ $CH_2$ $CH_2$ O THP |
| 46 | $CH_2$ $CH_2$ O $CH_3$ | $CH_2$ $CH_2$ $CH_2$ O CO NH Et |
| 47 | $CH_2$ $CH_2$ O $CH_3$ | $CH_2$ $CH_2$ $CH_2$ O CO NH Pr |
| 48 | $CH_2$ $CH_2$ O $CH_3$ | $CH_2$ $CH_2$ $CH_2$ O CO NH n-Bu |

| Bspl. | $R^1/R^2$ | $R^3/R^4$ |
|---|---|---|
| 49 | $CH_2$ $CH_2$ O $CH_3$ | $CH_2$ $CH_2$ $CH_2$ O CO NH c-Hex |
| 50 | $CH_2$ $CH_2$ OH | $CH_2$ $CH_2$ $CH_2$ O CO c-Hex |
| 51 | $CH_2$ $CH_2$ OH | $CH_2$ $CH_2$ $CH_2$ O CO c-Pent |
| 52 | $CH_2$ $CH_2$ OH | $CH_2$ $CH_2$ $CH_2$ O CO O Et |
| 53 | $CH_2$ $CH_2$ OH | $CH_2$ $CH_2$ $CH_2$ O CO O n-Bu |
| 54 | $CH_2$ $CH_2$ OH | $CH_2$ $CH_2$ $CH_2$ O CO O c-Hex |
| 55 | $CH_2$ $CH_2$ OH | $CH_2$ $CH_2$ $CH_2$ O CO O Ph |
| 56 | $CH_2$ $CH_2$ OH | $CH_2$ $CH_2$ $CH_2$ O CO (2-Me Ph) |
| 57 | $CH_2$ $CH_2$ OH | $CH_2$ $CH_2$ $CH_2$ O CO Ph |
| 58 | $CH_2$ $CH_2$ OH | $CH_2$ $CH_2$ $CH_2$ O $CH_2$ $CH_2$ $CH_2$ O- Me |
| 59 | $CH_2$ $CH_2$ OH | $CH_2$ $CH_2$ $CH_2$ O $CH_2$ $CH_2$ $CH_2$ O- Et |
| 60 | $CH_2$ $CH_2$ OH | $CH_2$ $CH_2$ $CH_2$ O $CH_2$ $CH_2$ $CH_2$ O-Ph |
| 61 | $CH_2$ $CH_2$ OH | $CH_2$ $CH_2$ $CH_2$ O Ph |
| 62 | $CH_2$ $CH_2$ OH | $CH_2$ $CH_2$ $CH_2$ O THP |
| 63 | $CH_2$ $CH_2$ OH | $CH_2$ $CH_2$ $CH_2$ O CO NH Et |
| 64 | $CH_2$ $CH_2$ OH | $CH_2$ $CH_2$ $CH_2$ O CO NH Pr |
| 65 | $CH_2$ $CH_2$ OH | $CH_2$ $CH_2$ $CH_2$ O CO NH n-Bu |
| 66 | $CH_2$ $CH_2$ OH | $CH_2$ $CH_2$ $CH_2$ O CO NH c-Hex |
| 67 | $CH_2$ $CH_2$ OH | $CH_2$ $CH_2$ $CH_2$ O CO NH Ph |

| Bspl. | $R^1/R^2$ | $R^3/R^4$ |
|---|---|---|
| 68 | $CH_2$ $CH_2$ $CH_2$ O $CH_3$ | $CH_2$ $CH_2$ $CH_2$ O CO c-Hex |
| 69 | $CH_2$ $CH_2$ $CH_2$ O $CH_3$ | $CH_2$ $CH_2$ $CH_2$ O CO c-Pen |
| 70 | $CH_2$ $CH_2$ $CH_2$ O $CH_3$ | $CH_2$ $CH_2$ $CH_2$ O CO O Eth |
| 71 | $CH_2$ $CH_2$ $CH_2$ O $CH_3$ | $CH_2$ $CH_2$ $CH_2$ O CO O n-Bu |
| 72 | $CH_2$ $CH_2$ $CH_2$ O $CH_3$ | $CH_2$ $CH_2$ $CH_2$ O CO O c-Hex |
| 73 | $CH_2$ $CH_2$ $CH_2$ O $CH_3$ | $CH_2$ $CH_2$ $CH_2$ O CO Ph |
| 74 | $CH_2$ $CH_2$ $CH_2$ O $CH_3$ | $CH_2$ $CH_2$ $CH_2$ O CO i-Pr |
| 75 | $CH_2$ $CH_2$ $CH_2$ O $CH_3$ | $CH_2$ $CH_2$ $CH_2$ O CO O Ph |
| 76 | $CH_2$ $CH_2$ $CH_2$ O $CH_3$ | $CH_2$ $CH_2$ $CH_2$ O $CH_2$ $CH_2$ O Me |
| 77 | $CH_2$ $CH_2$ $CH_2$ O $CH_3$ | $CH_2$ $CH_2$ $CH_2$ O $CH_2$ $CH_2$ O Et |
| 78 | $CH_2$ $CH_2$ $CH_2$ O $CH_3$ | $CH_2$ $CH_2$ $CH_2$ O $CH_2$ $CH_2$ O Ph |
| 79 | $CH_2$ $CH_2$ $CH_2$ O $CH_3$ | $CH_2$ $CH_2$ $CH_2$ O Ph |
| 80 | $CH_2$ $CH_2$ $CH_2$ O $CH_3$ | $CH_2$ $CH_2$ $CH_2$ O THP |
| 81 | $CH_2$ $CH_2$ $CH_2$ O $CH_3$ | $CH_2$ $CH_2$ $CH_2$ O CO NH Et |
| 82 | $CH_2$ $CH_2$ $CH_2$ O $CH_3$ | $CH_2$ $CH_2$ $CH_2$ O CO NH Pr |
| 83 | $CH_2$ $CH_2$ $CH_2$ O $CH_3$ | $CH_2$ $CH_2$ $CH_2$ O CO NH n-Bu |
| 84 | $CH_2$ $CH_2$ $CH_2$ O $CH_3$ | $CH_2$ $CH_2$ $CH_2$ O CO NH c-Hex |
| 85 | $CH_2$ $CH_2$ $CH_2$ OH | $CH_2$ $CH_2$ $CH_2$ O CO c-Hex |
| 86 | $CH_2$ $CH_2$ $CH_2$ OH | $CH_2$ $CH_2$ $CH_2$ O CO c-Pen |
| 87 | $CH_2$ $CH_2$ $CH_2$ OH | $CH_2$ $CH_2$ $CH_2$ O CO c-Et |
| 88 | $CH_2$ $CH_2$ $CH_2$ OH | $CH_2$ $CH_2$ $CH_2$ O CO C n-Bu |
| 89 | $CH_2$ $CH_2$ $CH_2$ OH | $CH_2$ $CH_2$ $CH_2$ O CO O c-Hex |

| Bspl. | $R^1/R^2$ | $R^3/R^4$ |
|---|---|---|
| 90 | $CH_2$ $CH_2$ $CH_2$ OH | $CH_2$ $CH_2$ $CH_2$ O CO O Ph |
| 91 | $CH_2$ $CH_2$ $CH_2$ OH | $CH_2$ $CH_2$ $CH_2$ O CO (2-Me Ph) |
| 92 | $CH_2$ $CH_2$ $CH_2$ OH | $CH_2$ $CH_2$ $CH_2$ O CO Ph |
| 93 | $CH_2$ $CH_2$ $CH_2$ OH | $CH_2$ $CH_2$ $CH_2$ O $CH_2$ $CH_2$ $CH_2$ O- Me |
| 94 | $CH_2$ $CH_2$ $CH_2$ OH | $CH_2$ $CH_2$ $CH_2$ O $CH_2$ $CH_2$ $CH_2$ O- Et |
| 95 | $CH_2$ $CH_2$ $CH_2$ OH | $CH_2$ $CH_2$ $CH_2$ O $CH_2$ $CH_2$ $CH_2$ O- Ph |
| 96 | $CH_2$ $CH_2$ $CH_2$ OH | $CH_2$ $CH_2$ $CH_2$ O Ph |
| 97 | $CH_2$ $CH_2$ $CH_2$ OH | $CH_2$ $CH_2$ $CH_2$ O THP |
| 98 | $CH_2$ $CH_2$ $CH_2$ OH | $CH_2$ $CH_2$ $CH_2$ O CO NH Et |
| 99 | $CH_2$ $CH_2$ $CH_2$ OH | $CH_2$ $CH_2$ $CH_2$ O CO NH Pr |
| 100 | $CH_2$ $CH_2$ $CH_2$ OH | $CH_2$ $CH_2$ $CH_2$ O CO NH n-Bu |
| 101 | $CH_2$ $CH_2$ $CH_2$ OH | $CH_2$ $CH_2$ $CH_2$ O CO NH c-Hex |
| 102 | $CH_2$ $CH_2$ $CH_2$ OH | $CH_2$ $CH_2$ $CH_2$ O CO NH Ph |
| 103 | $CH_2$ $CH_2$ $CH_2$ O CO c-Hex | $CH_2$ $CH_2$ O Me |
| 104 | $CH_2$ $CH_2$ $CH_2$ O CO c-Pen | $CH_2$ $CH_2$ O Me |
| 105 | $CH_2$ $CH_2$ $CH_2$ O CO O Et | $CH_2$ $CH_2$ O Me |
| 106 | $CH_2$ $CH_2$ $CH_2$ O CO O n-Bu | $CH_2$ $CH_2$ O Me |
| 107 | $CH_2$ $CH_2$ $CH_2$ O CO O c-Hex | $CH_2$ $CH_2$ O Me |
| 108 | $CH_2$ $CH_2$ $CH_2$ O CO O Ph | $CH_2$ $CH_2$ O Me |

| Bspl. | $R^1/R^2$ | $R^3/R^4$ |
|---|---|---|
| 109 | $CH_2$ $CH_2$ $CH_2$ O CO 2-Me Ph | $CH_2$ $CH_2$ O Me |
| 110 | $CH_2$ $CH_2$ $CH_2$ O CO Ph | $CH_2$ $CH_2$ O Me |
| 111 | $CH_2$ $CH_2$ $CH_2$ O $CH_2$ $CH_2$ $CH_2$ O-Me | $CH_2$ $CH_2$ O Me |
| 112 | $CH_2$ $CH_2$ $CH_2$ O THP | $CH_2$ $CH_2$ O Me |
| 113 | $CH_2$ $CH_2$ $CH_2$ O CO NH Et | $CH_2$ $CH_2$ O Me |
| 114 | $CH_2$ $CH_2$ $CH_2$ O CO NH Pr | $CH_2$ $CH_2$ O Me |
| 115 | $CH_2$ $CH_2$ $CH_2$ O CO NH n-Bu | $CH_2$ $CH_2$ O Me |
| 116 | $CH_2$ $CH_2$ $CH_2$ O CO NH c-Hex | $CH_2$ $CH_2$ O Me |
| 117 | $CH_2$ $CH_2$ $CH_2$ O CO NH Ph | $CH_2$ $CH_2$ O Me |
| 118 | $CH_2$ $CH_2$ $CH_2$ O CO c-Hex | $CH_2$ $CH_2$ $CH_2$ O Me |
| 119 | $CH_2$ $CH_2$ $CH_2$ O CO c-Pen | $CH_2$ $CH_2$ $CH_2$ O Me |
| 120 | $CH_2$ $CH_2$ $CH_2$ O CO O Et | $CH_2$ $CH_2$ $CH_2$ O Me |
| 121 | $CH_2$ $CH_2$ $CH_2$ O CO O n-Bu | $CH_2$ $CH_2$ $CH_2$ O Me |
| 122 | $CH_2$ $CH_2$ $CH_2$ O CO O c-Hex | $CH_2$ $CH_2$ $CH_2$ O Me |
| 123 | $CH_2$ $CH_2$ $CH_2$ O CO O Ph | $CH_2$ $CH_2$ $CH_2$ O Me |
| 124 | $CH_2$ $CH_2$ $CH_2$ O CO 2-Me Ph | $CH_2$ $CH_2$ $CH_2$ O Me |
| 125 | $CH_2$ $CH_2$ $CH_2$ O CO Ph | $CH_2$ $CH_2$ $CH_2$ O Me |
| 126 | $CH_2$ $CH_2$ $CH_2$ O $CH_2$ $CH_2$ $CH_2$ O Me | $CH_2$ $CH_2$ $CH_2$ O Me |
| 127 | $CH_2$ $CH_2$ $CH_2$ O THP | $CH_2$ $CH_2$ $CH_2$ O Me |
| 128 | $CH_2$ $CH_2$ $CH_2$ O CO NH Et | $CH_2$ $CH_2$ $CH_2$ O Me |

| Bspl. | $R^1/R^2$ | $R^3/R^4$ |
|---|---|---|
| 129 | $CH_2$ $CH_2$ $CH_2$ O CO NH Pr | $CH_2$ $CH_2$ $CH_2$ O Me |
| 130 | $CH_2$ $CH_2$ $CH_2$ O CO NH n-Bu | $CH_2$ $CH_2$ $CH_2$ O Me |
| 131 | $CH_2$ $CH_2$ $CH_2$ O CO NH c-Hex | $CH_2$ $CH_2$ $CH_2$ O Me |
| 132 | $CH_2$ $CH_2$ $CH_2$ = CO NH Ph | $CH_2$ $CH_2$ $CH_2$ O Me |
| 133 | $CH_2$ $CH_2$ $CH_2$ O CO c-Hex | $CH_2$ $CH_2$ $CH_2$ OH |
| 134 | $CH_2$ $CH_2$ $CH_2$ O CO c-Pen | $CH_2$ $CH_2$ $CH_2$ OH |
| 135 | $CH_2$ $CH_2$ $CH_2$ O CO O Et | $CH_2$ $CH_2$ $CH_2$ OH |
| 136 | $CH_2$ $CH_2$ $CH_2$ O CO O n-Bu | $CH_2$ $CH_2$ $CH_2$ OH |
| 137 | $CH_2$ $CH_2$ $CH_2$ O CO O c-Hex | $CH_2$ $CH_2$ $CH_2$ OH |
| 138 | $CH_2$ $CH_2$ $CH_2$ O CO O Ph | $CH_2$ $CH_2$ $CH_2$ OH |
| 139 | $CH_2$ $CH_2$ $CH_2$ O CO 2-Me Ph | $CH_2$ $CH_2$ $CH_2$ OH |
| 140 | $CH_2$ $CH_2$ $CH_2$ O CO Ph | $CH_2$ $CH_2$ $CH_2$ OH |
| 141 | $CH_2$ $CH_2$ $CH_2$ O $Ch_2$ $CH_2$ $CH_2$ O-Me | $CH_2$ $CH_2$ $CH_2$ OH |
| 142 | $CH_2$ $CH_2$ $CH_2$ O THP | $CH_2$ $CH_2$ $CH_2$ OH |
| 143 | $CH_2$ $CH_2$ $CH_2$ O CO NH Et | $CH_2$ $CH_2$ $CH_2$ OH |
| 144 | $CH_2$ $CH_2$ $CH_2$ O CO NH Pr | $CH_2$ $CH_2$ $CH_2$ OH |
| 145 | $CH_2$ $CH_2$ $CH_2$ O CO NH n-Bu | $CH_2$ $CH_2$ $CH_2$ OH |
| 146 | $CH_2$ $CH_2$ $CH_2$ O CO NH c-Hex | $CH_2$ $CH_2$ $CH_2$ OH |
| 147 | $CH_2$ $CH_2$ $CH_2$ O CO NH Ph | $CH_2$ $CH_2$ $CH_2$ OH |
| 148 | $CH_2$ $CH_2$ $CH_2$ O CO c-Hex | $CH_2$ $CH_2$ OH |
| 149 | $CH_2$ $CH_2$ $CH_2$ O CO c-Pen | $CH_2$ $CH_2$ OH |

| Bspl. | $R^1/R^2$ | $R^3/R^4$ |
|---|---|---|
| 150 | $CH_2\,CH_2\,CH_2$ O CO O Et | $CH_2\,CH_2$ OH |
| 151 | $CH_2\,CH_2\,CH_2$ O CO O n-Bu | $CH_2\,CH_2$ OH |
| 152 | $CH_2\,CH_2\,CH_2$ O CO O c-Hex | $CH_2\,CH_2$ OH |
| 153 | $CH_2\,CH_2\,CH_2$ O CO O Ph | $CH_2\,CH_2$ OH |
| 154 | $CH_2\,CH_2\,CH_2$ O CO 2-Me Ph | $CH_2\,CH_2$ OH |
| 155 | $CH_2\,CH_2\,CH_2$ O CO Ph | $CH_2\,CH_2$ OH |
| 156 | $CH_2\,CH_2\,CH_2$ O $CH_2\,CH_2\,CH_2$ O-Me | $CH_2\,CH_2$ OH |
| 157 | $CH_2\,CH_2\,CH_2$ O THP | $CH_2\,CH_2$ OH |
| 158 | $CH_2\,CH_2\,CH_2$ O CO NH Et | $CH_2\,CH_2$ OH |
| 159 | $CH_2\,CH_2\,CH_2$ O CO NH Pr | $CH_2\,CH_2$ OH |
| 160 | $CH_2\,CH_2\,CH_2$ O CO NH n-Bu | $CH_2\,CH_2$ OH |
| 161 | $CH_2\,CH_2\,CH_2$ O CO NH c-Hex | $CH_2\,CH_2$ OH |
| 162 | $CH_2\,CH_2\,CH_2$ O CO NH Ph | $CH_2\,CH_2$ OH |

Beispiel 163

Pyridin-2,4-dicarbonsäure-bis-N,N'-[2-(4-hydroxyphenyl)-ethyl]-amid

Analog Beispiel 8 aus Pyridin-2,4-dicarbonsäure-dimethylester und Tyramin farblose Kristalle; Fp. 165 - 166 °C
Summenformel: $C_{23}H_{23}N_3O_4$ (405)
MS: m/e = 40 (M + $H^+$).

Beispiel 164

Pyridin-2,4-dicarbonsäure-bis(N-methoxy-N-methylamid)

Zu 1,67 g (10 mMol) 2,4-Pyridindicarbonsäure suspendiert in 100 ml Dichlormethan, werden unter Rühren bei 20 °C 5,1 g (40 mM) N-Ethylmorpholin zugegeben, anschließend bei -15 °C mit 2,9 ml (20 ml) Chlorameisensäureisobutylester zugetropft und 20 min bei -10 °C gerührt. Dann gibt man portionsweise 1,95 (20 mM) N,O-Dimethylhydroxylaminhydrochlorid zu, rührt 1 Stunde bei - 15 °C und läßt über Nacht auf 20 °C erwärmen, versetzt die Mischung mit Wasser, extrahiert mit Dichlormethan und erhält nach säulenchromatographischer Reinigung des Rohprodukts an Kiesegel (Ethylacetat/Methanol = 10/1) 1,6 g der Titelverbindung als farbloses Öl,
Summenformel: $C_{11}H_{15}N_3O_4$ (253).

MS: m/e = 254 (M + H[7]).

Beispiel 165

Pyridin-2-carbonsäure-(2-methoxyethyl)amid-4-carbonsäure-(ethyloxy (N-tert.butyloxycarbonylglycyl))amid

0,8 g (3 mM) Pyridin-2-carbonsäure-(2-methoxyethyl)amid-4-carbonsäure-(2-hydroxyethyl)-amid (Verbindung aus Beispiel 6 d) werden in 25 ml wasserfreiem Acetonitril mit 525 mg (3 mM) N-Butyloxycarbonyl-Glycin, 0,4 ml (3 mM) N-Ethylmorpholin, 0,45 g (3,3 mM) N-Hydroxybenztriazol und 0,62 g (3 mM) N,N-Dicyclohexylcarbodiimid 20 Stunden bei 25 °C gerührt. Der angefallene N,N-Dicyclohexylharnstoff wird abgesaugt, mit Acetonitril gewaschen, eingeengt, in Dichlormethan aufgenommen, mit gesättigter wäßriger $NaHCO_3$-Lösung extrahiert, mit 10 %iger wäßriger Zitronensäure geschüttelt, getrocknet, vom Lösungsmittel befreit und der Rückstand an Kieselgel chromatographiert.
Summenformel: $C_{19}H_{28}N_4O_7$ (424),
MS: m/e = 425 (M + $H^+$).

Weitere Beispiele sind (Synthese erfolgt aus der in Beispiel 6 d) beschriebenen Verbindung Pyridin-2-carbonsäure-(-2-methoxyethyl)amid-4-carbonsäure-(2-hydroxyethyl)amid bzw. analogen Verbindungen durch Benzylierung)
Pyridin-2-carbonsäure-(2-methoxyethyl)amid-4-carbonsäure-(2-benzyloxyethyl)amid
Pyridin-2-carbonsäure-(2-hydroxyethyl)amid-4-carbonsäure-(2-benzyloxyethyl)amid
Pyridin-2-carbonsäure-(3-methoxypropyl)amid-4-carbonsäure-(2-benzyloxyethyl)amid
Pyridin-2-carbonsäure-(2-hydroxypropyl)amid-4-carbonsäure-(2-benzyloxyethyl)amid
Pyridin-2,4-dicarbonsäure-bis-N,N'-(benzyloxyethyl)amid
Pyridin-2,4-dicarbonsäure-(N,N'-benzyloxypropyl)-diamid
Pyridin-2-carbonsäure-(2-methoxyethyl)amid-4-carbonsäure-[2-(4-fluorbenzyloxy)ethyl]amid
Pyridin-2-carbonsäure-(2-hydroxyethyl)amid-4-carbonsäure-[2-(4-fluorbenzyloxy)ethyl]amid
Pyridin-2-carbonsäure-(3-hydroxypropyl)amd-4-carbonsäure-[2-(4-fluorbenzyloxy)ethyl]amid
Pyridin-2-carbonsäure-(2-methoxyethyl)amid-4-carbonsäure-[2-(4-methoxybenzyloxy)ethyl]amid
Pyridin-2-carbonsäure-[2-hydroxyethyl)amid-4-carbonsäure-[2-(4-methoxybenzyloxy)ethyl]amid
Pyridin-2-carbonsäure-(2-hydroxypropyl)amid-4-carbonsäure-[2-[4-methoxybenzyloxy)ethyl-amid
Pyridin-2-carbonsäure-(2-benzyloxyethyl)amid-4-carbonsäure-(2-hydroxyethyl)amid
Pyridin-2-carbonsäure-(2-benzyloxyethyl)amid-4-carbonsäure-(3-hydroxypropyl)amid
Pyrdin-2-carbonsäure-(2-benzyloxypropyl)amid-4-carbonsäure-(3-hydroxypropyl)amid
Pyridin-2-carbonsäure-[2-(4-chlorbenzyloxy)ethyl]amid-4-carbonsäure-[2-hydroxyethyl)amid.

Pyridin-2-carbonsäure-[2-(4-chlorbenzyloxy)ethyl]amid-4-carbonsäure-(3-hydroxypropyl)-amid
Pyridin-2-carbonsäinre-(2-methoxyethyl)amid-4-carbonsäure-(2-benzyloxypropyl)amid
Pyridin-2-carbonsäure-(2-hydroxyethyl)amid-4-carbonsäure-(2-benzyloxypropyl)amid
Pyridin-2-carbonsäure-(3-methoxypropyl)amid-4-carbonsäure-(2-benzyloxypropyl)amid
Pyridin-2-carbonsäure-(2-hydroxypropyl)amid-4-carbonsäure-(2-benzyloxypropyl)amid
Pyridin-2-carbonsäure-(2-methoxyethyl)amid-4-carbonsäure-[2-(4-fluorbenzyloxy)propyl)amid
Pyridin-2-carbonsäure-(2-hydroxyethyl)amid-4-carbonsäure-[2-(4-fluorbenzyloxy)propyl]amid
Pyridin-2-carbonsäure-(3-hydroxypropyl)amid-4-carbonsäure-[2-(4-fluorbenzyloxy)propyl]amid
Pyridin-2-carbonsäure-(2-methoxyethyl)amid-4-carbonsäure-[2-(4-methoxybenzyloxy)propyl]-amid
Pyridin-2-carbonsäure-(2-hydroxyethyl)amid-4-carbonsäure-[2-(4-methoxybenzyloxy)propyl]-amid
Pyridin-2-carbonsäure-(2-hydroxypropyl)amid-4-carbonsäure-[2-(4-methoxybenzyloxy)propyl]-amid
Pyrdin-2-carbonsäure-(2-benzyloxypropyl)amid-4-carbonsäure-(2-hydroxyethyl)amd
Pyridin-2-carbonsäure-(2-benzyloxypropyl)amid-4-carbonsäure-(3-hydroxypropyl)amid
Pyridin-2-carbonsäure-[2-(4-chlorbenzyloxy)propyl]amid-4-carbonsäure-(2-hydroxyethyl)amid.

Pyridin-2-carbonsäure-[2-(4-chlorbenzyloxy)propyl]amid-4-carbonsäure-(3-hydroxypropyl)-amid
Pyridin-2-carbonsäure-(2-methoxyethyl)amid-5-carbonsäure-(2-benzyloxyethyl)amid
Pyridin-2-carbonsäure-(2-hydroxyethyl)amid-5-carbonsäure-(2-benzyloxyethyl)yamid

Pyridin – 2 – carbonsäure – (3 – methoxypropyl)amid – 5 – carbonsäure – (2 – benzyloxyethyl)amid
Pyridin – 2 – carbonsäure – (2 – hydroxypropyl)amid – 5 – carbonsäure – (2 – benzyloxyethyl)amid
Pyridin – 2,5 – dicarbonsäure – bis – N,N' – (benzyloxyethyl)amid
Pyridin – 2,5 – dicarbonsäure – (N,N' – benzyloxypropy) – diamid
Pyridin – 2 – carbonsäure – (2 – methoxyethyl)amid – 5 – carbonsäure – [2 – (4 – fluorbenzyloxy)ethyl]amid
Pyrdin – 2 – carbonsäure – (2 – hydroxyethyl)amid – 5 – carbonsäure – [2 – (4 – fluorbenzyloxy)ethyl]amid
Pyridin – 2 – carbonsäure – (3 – hydroxypropyl)amid – 5 – carbonsäure – [2 – (4 – fluorbenzyloxy)ethyl]amid
Pyridin – 2 – carbonsäure – (2 – methoxyethyl)amid – 5 – carbonsäure – [2 – (4 – methoxybenzyloxy)ethyl]amid
Pyridin – 2 – carbonsäure – (2 – hydroxyethyl)amid – 5 – carbonsäure – [2 – (4 – methoxybenzyloxy)ethyl]amid
Pyridin – 2 – carbonsäure – (2 – hydroxypropyl)amid – 5 – carbonsäure – [2 – (4 – methoxybenzyloxy)ethyl] – amid
Pyridin – 2 – carbonsäure – (2 – benzyloxyethyl)amid – 5 – carbonsäure(2 – hydroxyethyl)amid
Pyridin – 2 – carbonsäure – (2 – benzyloxyethyl)amid – 5 – carbonsäure – (3 – hydroryprop y)amid
Pyridin – 2 – carbonsäure – (2 – benzyloxypropyl)amid – 5 – carbonsäure – (3 – hydroxypropyl)amid
Pyridin – 2, – carbonsäure – [2 – (4 – chlorbenzyloxy)ethyl]amid – 5 – carbonsäure – (2 – hydroxyethyl)amid.
Pyridin – 2 – carbonsäure – [2 – (4 – chlorbenzyloxy)ethyl]amid – 5 – carbonsäure – (3 – hydroxypropyl) – amid

## Patentansprüche

1. Verbindungen der allgemeinen Formel I

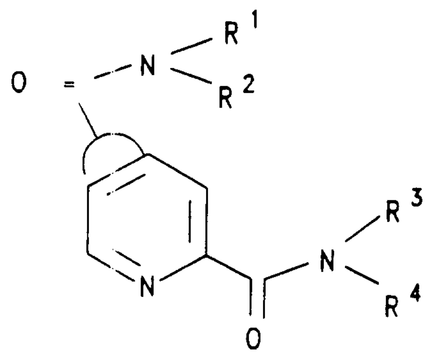

( I )

worin
$R^1$, $R^2$, $R^3$ und $R^4$ gleich oder verschieden sind und
A einen verzweigten oder unverzweigten, aliphatischen oder cycloaliphatischen $(C_1-C_{12})$ – Alkylrest, $(C_1-C_{12})$ – Alkenylrest oder einen $(C_1-C_{12})$ – Alkinylrest bedeutet, der einfach oder mehrfach, vorzugsweise einfach oder zweifach substituiert ist mit
einem $(C_1-C_8)$ – Alkoxycarbonyloxy, $(C_1-C_8)$ – Alkoxy – $(C_1-C_8)$ – alkoxycarbonyloxy, $(C_6-C_{12})$ – Aryloxycarbonyloxy, $(C_7-C_{11})$ – Aralkyloxycarbonyloxy, $(C_7-C_{11})$ – Aralkylcarbonyloxy, Cinnamoyl, Cinnamoyloxy, $(C_6-C_{12})$ – Arylcarbonyloxy, $(C_3-C_8)$ – Alkenylcarbonyloxy, $(C_3-C_8)$ – Alkinylcarbonyloxy, $(C_3-C_8)$ – Cycloalkylcarbonyloxy, $(C_1-C_{12})$ – Alkoxy – $(C_1-C_{12})$ – alkoxy, $(C_1-C_{12})$ – Alkoxyamino, $(C_1-C_{12})$ – Alkoxy – N $(C_1-C_6)$ – alkylamino, $(C_1-C_{12})$ – Alkoxy – N,N $(C_1-C_6)$ – dialkylamino, Carbamoyloxy, N – $(C_1-C_8)$ – Alkylcarbamoyloxy, N,N – Di – $(C_1-C_8)$ – alkylcarbamoyl, N – $(C_3-C_8)$ – Cycloalkylcarbamoyl, N – $(C_6-C_{12})$ – Arylamino, N – $(C_7-C_{11})$ – Aralkylamino, N – Alkyl – Aralkylamino, N – Alkyl – Arylamino, $(C_3-C_8)$ – Cycloalkanoylamino, $(C_1-C_8)$ – Alkanoylamino, $(C_6-C_{12})$ – Aroylamino, $(C_7-C_{11})$ – Aralkanoylamino, $(C_1-C_8)$ – Alkanoyl – $(C_1-C_8)$ – alkylamino, $(C_3-C_8)$ – Cycloalkanoyl – $(C_1-C_8)$ – alkylamino, $(C_6-C_{12})$ – Aroyl – $(C_1-C_8)$ – alkylamino, $(C_7-C_{11})$ – Aralkanoyl – $(C_1-C_8)$ – alkylamino, $(C_1-C_8)$ – Alkymercapto, $(C_1-C_8)$ – Alkylsulfinyl, $(C_1-C_8)$ – Alkylsulfonyl, $(C_1-C_8)$ – Alkylcarbonyl, $(C_3-C_8)$ – Cycloalkylcarbonyl, Nitro, Trifluormethyl, Phenylmercapto, Phenylsulfonyl, Phenylsulfinyl, Sulfamoyl, N – $(C_1-C_6)$ – Alkylsulfamoyl, N,N – Di – $(C_1-C_6)$ – alkylsulfamoyl, $(C_1-C_8)$ – Alkyl – sulfonamido, Arylsulfonamido, wobei die in vorstehenden Substituenten vorhandenen Aryl – und Aralkylreste auch heterocyclischer Natur sein können und/oder, wie auch Alkyl, mit 1, 2, 3, 4 oder 5 gleichen oder verschiedenen Substituenten aus der Reihe Halogen, Cyano, Nitro, Trifluormethyl, $(C_1-C_6)$ – Alkyl Hydroxy, $(C_1-C_6)$ – Hydroxyalkyl, $(C_1-C_6)$ – Alkoxy, – O – [$CH_2$ –

$]_xC_fH_{(2f+1-g)}F_g$, $-OCF_2Cl$, $-O-CF_2-CHFCl$,Trifluormethyl ($C_1-C_6$)-Alkylmercapto ($C_1-C_6$)-Alkylsulfinyl, ($C_1-C_6$)-Alkylsulfonyl, ($C_1-C_6$)-Alkylcarbonyl, ($C_1-C_6$)-Alkoxycarbonyl, Carbamoyl, N-($C_1-C_4$)-Alkylcarbamoyl, N,N-Di-($C_1-C_4$)-alkylcarbamoyl, ($C_1-C_6$)-Alkylcarbonyloxy, ($C_3-C_8$)-Cycloalkyl, Phenyl, Benzyl, Phenoxy, Benzyloxy, NR'-R'', Phenylmercapto, Phenylsulfonyl, Phenylsulfinyl, Sulfamoyl, N-($C_1-C_4$)-Alkylsulfamoyl oder N,N-Di-($C_1-C_4$)-alkylsulfamoyl, insbesondere bis zu mit 3 der vorstehend genannten gleichen oder verschiedenen Substituenten substituiert sind und eine $CH_2$-Gruppe der Alkylkette gegebenenfalls durch O, S, SO, $SO_2$ oder NR' ersetzt ist, oder mit

einem substituierten ($C_6-C_{12}$)-Arylrest oder Heteroarylrest, der 1, 2, 3, 4 oder 5 gleiche oder verschieoene Substituenten aus der Reihe Hydroxy, Trifluormethyl, ($C_1-C_6$)-Hydroxyalkyl, $-O-[CH_2-]_xC_fH_{(2f+1-g)}F_g$, $-OCF_2Cl$, $-OCF_2-CHFCl$, ($C_1-C_6$)-Alkylmercapto, ($C_1-C_6$)-Alkylsulfinyl, ($C_1-C_6$)-Alkylsulfonyl, ($C_1-C_6$)-Alkylcarbonyl, ($C_1-C_6$)-Alkoxycarbonyl, Carbamoyl, N-($C_1-C_4$)-Alkylcarbamoyl, N,N-Di-($C_1-C_4$)-alkylcarbamoyl, ($C_1-C_6$)-Alkylcarbonyloxy, ($C_3-C_8$)-Cycloalkyl, Phenyl, Benzyl, Phenoxy, Benzyloxy, NR'-R'', Phenylmercapto, Phenylsulfonyl, Phenylsulfinyl, Sulfamoyl, N-($C_1-C_4$)-Alkylsulfamoyl, N,N-Di-($C_1-C_4$)-alkylsulfamoyl, ($C_1-C_8$)-Alkoxycarbonyloxy, ($C_1-C_8$)-Alkoxy-($C_1-C_8$)-alkoxycarbonyloxy, ($C_6-C_{12}$)-Aryloxycarbonyloxy, ($C_7-C_{11}$)-Aralkyloxycarbonyloxy, ($C_7-C_{11}$)-Aralkylcarbonyloxy, Cinnamoyl, Cinnamoyloxy, ($C_6-C_{12}$)-Arylcarbonyloxy, ($C_3-C_8$)-Alkenylcarbonyloxy, ($C_3-C_8$)-Alkinylcarbonyloxy, ($C_3-C_8$)-Cycloalkylcarbonyloxy, ($C_1-C_{12}$)-Alkoxy-($C_1-C_{12}$)-alkoxy, ($C_1-C_{12}$)-Alkoxy-amino, ($C_1-C_{12}$)-Alkoxy-N ($C_1-C_6$)-alkylamino, ($C_1-C_{12}$)-Alkoxy-N,N($C_1-C_6$)-dialkylamino, Carbamoyloxy, N-($C_1-C_8$)-Alkylcarbamoyloxy, N,N-Di-($C_1-C_8$)-alkylcarbamoyl, N($C_3-C_8$)-Cycloalkylcarbamoyl, N-($C_6-C_{12}$)-Arylamino, N-($C_7-C_{11}$)-Aralkylamino, N-Alkyl-Aralkylamino, N-Alkyl-Arylamino, ($C_3-C_8$)-Cycloalkanoylamino, ($C_1-C_8$)-Alkanoylamino, ($C_6-C_{12}$)-Aroylamino, ($C_7-C_{11}$)-Aralkanoylamino, ($C_1-C_8$)-Alkanoyl-($C_1-C_8$)-alkylamino, ($C_3-C_8$)-Cycloalkanoyl-($C_1-C_8$)-alkylamino, ($C_6-C_{12}$)-Aroyl-($C_1-C_8$)-alkylamino, ($C_7-C_{11}$)-Aralkanoyl-($C_1-C_8$)-alkylamino,($C_1-C_8$)-Alkylmercapto, ($C_1-C_8$)-Alkylsulfinyl, ($C_1-C_8$)-Alkylsulfonyl, ($C_1-C_8$)-Alkylcarbonyl,($C_3-C_8$)-Cycloalkylcarbonyl, Nitro, Trifluormethyl, Phenylmercapto, Phenylsulfonyl, Phenylsulfinyl, Sulfamoyl, N-($C_1-C_6$)-Alkylsulfamoyl, N,N-Di-($C_1-C_6$)-alkylsulfamoyl, ($C_1-C_8$)-Alkyl-sulfonamido und Arylsulfonamido trägt, wobei die in vorstehenden Substituenten vorhandenen Aryl- und Aralkylreste auch heterocyclischer Natur sein können und/oder, wie auch Alkyl, mit 1, 2, 3, 4 oder 5 gleichen oder verschiedenen Substituenten aus der Reihe Halogen, Cyano, Nitro, Trifluormethyl, ($C_1-C_6$)-Alkyl Hydroxy, ($C_1-C_6$)-Hydroxyalkyl, ($C_1-C_6$)-Alkoxy, substituiert sein können, oder mit

einem substituierten ($C_6-C_{12}$)-Aryloxyrest, ($C_7-C_{11}$)-Aralkyloxyrest oder Heteroaryloxyrest, der 1, 2, 3, 4 oder 5 gleiche oder verschiedene Substituenten aus der Reihe Hydroxy, Halogen, Cyano, Nitro, Trifluormethyl, ($C_1-C_6$)-Hydroxyalkyl, ($C_1-C_6$)Alkoxy, $[CH_2-]_xC_fH_{(2f+1-g)}F_g$, $-OCF_2-CHFCl$, ($C_1-C_6$)-Alkylmercapto, ($C_1-C_6$)-Alkylsulfinyl, ($C_1-C_6$)-Alkylsulfonyl, ($C_1-C_6$)-Alkylcarbonyl, ($C_1-C_6$)-Alkoxycarbonyl, Carbamoyl, N-($C_1-C_4$)-Alkylcarbamoyl, N,N-Di-($C_1-C_4$)-alkylcarbamoyl, ($C_1-C_6$)-Alkylcarbonyloxy, ($C_3-C_8$)-Cycloalkyl, Carboxyl, Phenyl, Benzyl, Phenoxy, Benzyloxy, NR'-R'', Phenylmercapto, Phenylsulfonyl, Phenylsulfinyl, Sulfamoyl, N-($C_1$-$C_4$)-Alkylsulfamoyl, N,N-Di-($C_1-C_4$)-alkylsulfamoyl Aminoalkyl, N-($C_1-C_8$)-alkylamino-($C_1-C_{12}$)-alkyl oder N-Di-($C_1-C_8$)-alkylamino-($C_1$-$C_{12}$)-alkyl trägt, insbesondere bis zu mit 3 der vorstehend genannten gleichen oder verschiedenen Substituenten substituiert ist und eine $CH_2$-Gruppe der Alkylkette gegebenenfalls durch O, S, SO, $SO_2$ oder NR' ersetzt ist, oder mit

einem Rest der allgemeinen Formel II

$$-O-R^5 \quad (II),$$

worin

$R^5$ eine über ihren Acylrest gebundene Aminosäure ihr, Derivat oder eine Alkoholschutzgruppe bedeutet,

B einen substituierten ($C_6-C_{12}$)Arylrest, ($C_7-C_{11}$)Aralkylrest oder einen Heteroarylrest bedeutet, der einfach oder mehrfach, vorzugsweise ein-oder zweifach substituiert ist mit

Hydroxy, Amino, ($C_1-C_8$)-Alkoxycarbonyl, ($C_1-C_8$)-Alkylcarbonyloxy, ($C_1-C_8$)-Alkylamino, Di-($C_1-C_8$)-alkylamino, ($C_1-C_6$)-Hydroxyalkyl, $-O-[CH_2-]_xC_fH_{(2f+1-g)}F_g$, $-OCF_2Cl$, $-OCF_2-CHFCl$, ($C_1-C_8$)-Alkoxycarbonyl, Carbamoyl, N-($C_1-C_4$)-Alkylcarbamoyl, N,N-Di-($C_1-C_8$)-alkylcarbamoyl, ($C_1-C_8$)-Alkylcarbonyloxy, ($C_3-C_8$)-Cycloalkyl, Phenyl, Benzyl, Phenoxy, Benzyloxy, Aminoalkyl, N-($C_4-C_8$)-alkylamino ($C_1-C_{12}$)-alkyl oder N-Di-($C_1-C_8$)-alkylamino-($C_1-C_{12}$)-alkyl, ($C_1-C_8$)-Alkorycarbonyloxy, ($C_1-C_8$)-Alkoxy-($C_1-C_8$)-alkoxycarbonyloxy, ($C_6-C_{12}$)-

Aryloxycarbonyloxy, $(C_7-C_{11})$-Aralkyloxycarbonyloxy, $(C_7-C_{11})$-Aralkylcarbonyloxy, Cinnamoyl, Cinnamoyloxy, $(C_6-C_{12})$-Arylcarbonyloxy, $(C_3-C_8)$-Alkenylcarbonyloxy, $(C_3-C_8)$-Alkinylcarbonyloxy, $(C_3-C_8)$-Cycloalkylcarbonyloxy, $(C_1-C_{12})$-Alkoxy-$(C_1-C_{12})$-alkoxy, $(C_1-C_{12})$-Alkoxyamino, $(C_1-C_{12})$-Alkoxy-N $(C_1-C_6)$-alkylamino, $(C_1-C_{12})$-Alkoxy-N,N $(C_1-C_6)$-dialkylamino, Carbamoyloxy, N-$(C_1-C_8)$-Alkylcarbamoyloxy, N,N-Di-$(C_1-C_8)$-alkylcarbamoyl, N-$(C_3-C_8)$-Cycloalkylcarbamoyl, N-$(C_6-C_{12})$-Arylamino, N-$(C_7-C_{11})$-Aralkylamino, N-Alkyl-Aralkylamino, N-Alkyl-Arylamino, $(C_3-C_8)$-Cycloalkanoylamino, $(C_1-C_8)$-Alkanoylamino, $(C_6-C_{12})$-Aroylamino, $(C_7-C_{11})$-Aralkanoylamino, $(C_1-C_8)$-Alkanoyl-$(C_1-C_8)$-alkylamino, $(C_3-C_8)$-Cycloalkanoyl-$(C_1-C_8)$-alkylamino, $(C_6-C_{12})$-Aroyl-$(C_1-C_8)$-alkylamino, $(C_7-C_{11})$-Aralkanoyl-$(C_1-C_8)$-alkylamino, $(C_1-C_8)$-Alkylmercapto, $(C_1-C_8)$-Alkylsulfinyl, $(C_1-C_8)$-Alkylsulfonyl, $(C_1-C_8)$-Alkylcarbonyl,$(C_3-C_8)$-Cycloalkylcarbony, Nitro, Trifluormethyl, Phenylmercapto, Phenylsulfonyl, Phenylsulfinyl, Sulfamoyl, N-$(C_1-C_6)$-Alkylsulfamoyl, N,N-Di-$(C_1-C_6)$-alkylsulfamoyl, $(C_1-C_8)$-Alkyl-sulfonamido, Arylsulfonamido.

C einen substituierten $(C_1-C_{12})$Alkoxyrest, $(C_3-C_8)$-Cycloalkoxy, $(C_6-C_{12})$-Aryloxyrest oder einen $(C_7-C_{11})$-Aralkyloxyrest bedeuten, der einfach oder mehrfach, vorzugsweise ein- und zweifach substituiert ist mit

Halogen, Trifluormethyl, $(C_1-C_6)$-Alkoxy, Hydroxy, $(C_1-C_6)$-Hydroxyalkyl, NR'R'' oder Cyano, wobei jeweils

R' und R'' gleich oder verschieden sind und Wasserstoff, $(C_6-C_{12})$-Aryl, $(C_1-C_8)$-Alkyl, $(C_1-C_8)$-Alkylcarbonyl, $(C_7-C_{11})$Aralkylcarbonyl oder $(C_6-C_{12})$-Arylcarbonyl bedeuten oder mit dem Stickstoff einen gesättigten heterocyclischen Ring bilden, vorzugsweise einen 5- oder 6-gliedrigen Ring, und die genannten Reste $R^1$, $R^2$, $R^3$ und $R^4$ in Kombination mit einem

$(C_1-C_{12})$-Alkylrest, der einfach oder mehrfach, vorzugsweise ein- und zweifach, mit Wasserstoff, Halogen, Hydroxy, Cyano, Amino, Carboxyl, $(C_1-C_4)$-Alkoxy, $(C_1-C_4)$-Alkoxycarbonyl, $(C_1-C_4)$-Alkylcarbonyloxy, $(C_1-C_4)$ Alkyl- oder $(C_1-C_4)$-Dialkylamino oder einem Phenylring, der ein-, zwei- oder dreifach mit den Resten Halogen, Nitro, $(C_1-C_4)$-Alkyl oder $(C_1-C_4)$-Alkoxy substituiert ist, auftreten können, oder in Kombination mit einem

Aryl- oder Heteroarylrest, der seinerseits gegebenenfalls mit Halogen, Nitro, Cyano, $(C_1-C_4)$-Alkyl oder $(C_1-C_4)$-Alkoxy 1-, 2- oder 3-fach substituiert sein kann, zuzüglich aller Derivate, die in ihren Amino- oder Hydroxygruppen eine entsprechende Schutzgruppe aufweisen sowie die physiologisch wirksame Salze und

n = 0 der 1,
f = 1 bis 8, vorzugsweise 1 bis 5,
g = 0,1 bis (2f+1),
x 0, 1, 2 oder 3, vorzugsweise 0 oder 1 ist.

**2.** Verbindungen nach Anspruch 1, in denen $R^1$ und/oder $R^3$ Wasserstoff oder Methyl bedeuten und $R^2$ und/oder $R^4$ die in Anspruch 1 angebene Bedeutungen haben.

**3.** Verbindungen nach den Ansprüchen 1 oder 2, in denen $R^1$ und/oder $R^3$ Wasserstoff und $R^2$ und/oder $R^4$

A einen verzweigten oder unverzweigten $(C_1-C_{12})$-Alkylrest bedeutet, der einfach oder mehrfach substituiert ist mit

$(C_1-C_8)$-Alkoxycarbonyloxy, $(C_1-C_8)$-Alkoxy-$(C_1-C_8)$-alkoxycarbonyloxy, $(C_6-C_{12})$-Aryloxycarbonyloxy, $(C_7-C_{11})$-Aralkyloxycarbonyloxy, $(C_7-C_{11})$-Aralkylcarbonyloxy, $(C_7-C_{11})$-Arylcarbonyloxy, $(C_3-C_8)$-Cycloalkylcarbonyloxy, $(C_1-C_{12})$-Alkoxy-$(C_1-C_{12})$-alkoxy, Carbamoyloxy, N-$(C_1-C_8)$-Alkylcarbamoyloxy, N,N-Di $(C_1-C_8)$-alkylcarbamoyl, N-$(C_3-C_8)$-Cycloalkylcarbamoyl, N-$(C_7-C_{11})$-Aralkylcarbamoyloxy, N $(C_6-C_{12})$-Arylcarbamoyloxy, wobei die in vorstehenden Substituenten vorhandenen Aryl- und Aralkylreste auch heterocyclischer Natur sein können und/oder wie auch Alkyl mit 1 oder 2 gleichen oder verschiedenen Substituenten aus der Reihe Halogen, Trifluormethyl, Hydroxy, $(C_1-C_3)$-Alkyl, $(C_1-C_3)$-Hydroxyalkyl, $(C_1-C_9)$-Alkoxy, $-O-[CH_2-]_xC_fH_{(2f+1-g)}F_g$, $-OCF_2Cl$, $-O-CF_2-CHFCl$, $-(C_1-C_3)$-Alkoxycarbonyl, Carbamoyl, $(C_1-C_6)$-Alkylcarbonyloxy, $(C_3-C_8)$-Cycloalkyl, Phenyl, Benzyl, Phenoxy, Benzyloxy, substituiert sind, oder mit einem substituierten $(C_6-C_{12})$-Arylrest oder Heteroarylrest, der 1 oder 2 gleiche oder verschiedene Substituenten aus der Reihe Hydroxy, Trifluormethyl, $(C_1-C_3)$-Hydroxyalkyl, $(C_1-C_3)$-Alkoxycarbonyl, Carbamoyl, NR'R'', N-$(C_1-C_4)$-Alkylcarbamoyl, N,N-Di-$(C_1-C_4)$-alkylcarbamoyl, $(C_1-C_3)$-Alkylcarbonyloxy, Aminoalkyl oder N-$(C_1-C_4)$-alkylamino-$(C_1-C_6)$-alkyl trägt, wobei R' und R'' gleich oder verschieden sind und Wasserstoff, $(C_6-C_{12})$-Aryl, oder $(C_1-C_4)$-Alkyl bedeuten,

oder mit
einem substituierten $(C_6-C_{10})$-Aryloryrest oder $(C_7-C_{11})$-Aralkyloxyrest, der 1 oder 2 gleiche oder verschiedene Substituenten aus der Reihe Hydroxy, Halogen, Trifluormethyl, $(C_1-C_3)$-Alkyl, $(C_1-C_3)$-Hydroxyalkyl, $(C_1-C_3)$-Alkoxy, $(C_1-C_3)$-Alkylmercapto, $(C_1-C_3)$-Alkylsulfinyl, $(C_1-C_3)$-Alkylsulfonyl, $(C_1-C_3)$-Alkylcarbonyl, $(C_1-C_3)$-Alkorycarbonyl, Carbamoyl, N-$(C_1-C_4)$-Alkylcarbamoyl, N,N-Di-$(C_1-C_4)$-alkylcarbamoyl, $(C_1-C_3)$-Alkylcarbonyloxy, NR'R'', wobei R' und R'' gleich oder verschieden sind und Wasserstoff, $(C_6-C_{10})$-Aryl oder $(C_1-C_4)$-Alkyl bedeuten, oder mit einem Rest der allgemeinen Formel II

$$-O-R^5 \qquad (II)$$

worin
$R^5$ eine über ihren Acylrest gebundene Aminosäure oder ein Derivat hiervon bedeutet,
B einen $(C_6-C_{12})$Aryl- oder $(C_7-C_{11})$Aralkylrest, vorzugsweise Phenyl, Benzyl und Phenethyl, bedeuten, welche einfach substituiert sind mit Hydroxy, $(C_1-C_4)$-Alkylcarbonyloxy, $(C_1-C_4)$-Alkoxycarbonyl, $(C_1-C_4)$-Hydroxyalkyl, Amino, $(C_1-C_5)$-Alkylamino, Di-$(C_1-C_5)$alkylamino, $(C_1-C_5)$-Alkanoylamino, Carbamoyl, N-$(C_1-C_4)$-Alkylcarbamoyl, N,N-Di-$(C_1-C_4)$-alkylcarbamoyl, Carbamoyl, N-$(C_1-C_4)$-Alkylcarbamoyloxy, N,N-Di-$(C_1-C_4)$-alkylcarbamoyloxy, oder
C einen $(C_1-C_6)$Alkoxyrest, $(C_3-C_8)$-Cycloalkoxyrest, $(C_6-C_{12})$-Aryloxyrest und $(C_7-C_{11})$-Aralkyloxyrest bedeuten,

n = 0 oder 1,
f = 1 bis 8, vorzugsweise 1 bis 5,
g = 0, 1 bis (2f + 1),
x 0, 1, 2 oder 3, vorzugsweise 0 oder 1 ist und

wobei die genannten Reste $R^1$, $R^2$, $R^3$ und $R^4$ in Kombination mit einem
$(C_1-C_{12})$-Alkylrest, der einfach oder mehrfach, vorzugsweise ein- oder zweifach, mit Wasserstoff, Halogen, Hydroxy, Amino, $(C_1-C_4)$-Alkoxy, $(C_1-C_4)$ Alkoxycarbonyl, $(C_1-C_4)$ Alkyl- oder $(C_1-C_4)$ Dialkylamino oder einem Phenylring, der ein-, zwei- oder dreifach mit den Resten Halogen, Nitro, $(C_1-C_4)$-Alkyl oder $(C_1-C_4)$-Alkoxy substituiert ist, auftreten können und auch in Kombination mit einem
Aryl- oder Heteroarylrest, der seinerseits gegebenenfalls mit Halogen, $(C_1-C_4)$-Alkyl oder $(C_1-C_4)$-Alkoxy 1- oder 2-fach substituiert sein kann, zuzüglich alle Derivate, die in der entsprechenden Amino- oder Hydroxygruppe eine Schutzgruppe aufweisen, sowie die physiologisch wirksamen Salze.

**4.** Verbindungen nach den Ansprüche 1 bis 3, in denen $R^1$ und/oder $R^3$ Wasserstoff und $R^2$ und/oder $R^4$
A einen unverzweigten $(C_1-C_{12})$-Alkylrest bedeutet, der einfach substituiert ist mit
$(C_1-C_8)$-Alkoxycarbonylory, $(C_1-C_8)$-Alkoxy-$(C_1-C_8)$-alkoxycarbonyloxy, $(C_6-C_{12})$-Aryloxycarbonyloxy, $(C_7-C_{11})$-Aralkyloxycarbonyloxy, $(C_7-C_{11})$-Aralkylcarbonyloxy, $(C_6-C_{12})$-Arylcarbonyloxy, $(C_3-C_8)$-Cycloalkylcarbonyloxy, $(C_1-C_{12})$-Alkoxy-$(C_1-C_{12})$-alkoxy, $(C_1-C_{12})$-Alkoxy, -amino, $(C_1-C_{12})$-Alkoxy-N-$(C_1-C_6)$-alkylamino, $(C_1-C_{12})$-Alkoxy-N,N-$(C_1-C_6)$-dialkylamino, N,N-Di-$(C_1-C_8)$-alkylcarbamoyl, N-$(C_3-C_8)$-Cycloalkylcarbamoyl, N$(C_7-C_{11})$-Aralkylcarbonyloxy, N-$(C_6-C_{12})$-Arylcarbanoyloxy, $(C_1-C_5)$-Alkanoylamino, $(C_3-C_6)$-Cycloalkanoylamino, $(C_6-C_{12})$-Aroylamino, $(C_7-C_{11})$-Aralkamoylamino, wobei Alkyl, Aryl, Aryloxy, Aralkyl oder Aralkyloxy, ihrerseits substituiert sind mit Hydroxy, Halogen, insbesondere Fluor, $(C_1-C_3)$Alkyl, $(C_1-C_3)$Alkoxy, oder mit
einem einfach mit einer Hydroxy-Gruppe substituierten Phenylrest, einem substituierten Phenoxy- oder Benzyloxyrest, substituiert mit Hydroxy, Halogen, $(C_1-C_4)$ Alkoxy, oder mit
einem Rest der allgemeinen Formel II

$$-O-R^5 \qquad (II)$$

worin $R^5$ eine über ihren Acylrest gebundene Aminosäure oder ihr an der Aminogruppe substituiertes Derivat bedeutet,
B einen $(C_6-C_{12})$-Aryl- oder $(C_7-C_{11})$-Aralkylrest, vorzugsweise Phenyl, Benzyl und Phenethyl, bedeuten, welcher einfach substituiert ist mit Hydroxy und
C Methoxy bedeutet.

**5.** Verbindungen nach den Ansprüchen 1 bis 4, dadurch gekennzeichnet, daß als Rest ($C_7-C_{11}$)-Aralkyloxy Verbindung der Formel III

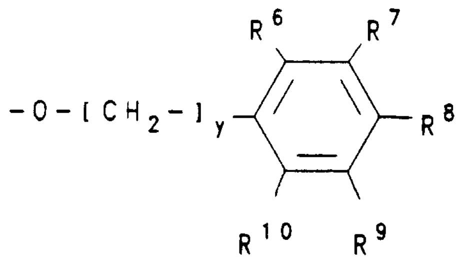

( I I I )

worin $R^6$, $R^7$, $R^8$ und $R^{10}$ gleich oder verschieden sind und Wasserstoff, Halogen, Cyano, Nitro, Trifluormethyl, ($C_1-C_6$)-Alkyl, ($C_1-C_6$)-Alkoxy, $-O-[CH_2-]_xC_fH_{(2f+1-g)}F_g$, $-OCF_2Cl$, $-O-CF_2-CHFCl$, ($C_1-C_6$)-Alkylmercapto, ($C_1-6_6$)-Alkylsulfinyl, ($C_1-C_6$)-Alkylsulfonyl, ($C_1-C_6$)-Alkylcarbonyl, ($C_1-C_6$)-Alkoxycarbonyl, Carbamoyl, N-($C_1-C_4$)-Alkylcarbamoyl, N,N-Di-($C_1-C_4$)-alkylcarbamoyl, ($C_1-C_6$)-Alkylcarbonyloxy, ($C_3-C_8$)-Cycloalkyl, Phenyl, Benzyl, Phenoxy, Benzyloxy, NR'R'', wie Amino, Anilino, N-Methylanilino, Phenylmercapto, Phenylsulfonyl, Phenylsulfinyl, Sulfamoyl, N-($C_1-C_4$)-Alkylsulfamoyl oder N-N-Di-($C_1-C_4$)-alkylsulfamoyl bedeuten, oder zwei benachbarte Substituenten gemeinsam eine Kette $-[CH_2-]_n$ oder $-CH=CH-CH=CH-$bedeuten, wobei eine $CH_2$-Gruppe der Kette gegebenenfalls durch O, S, SO, $SO_2$ oder NR' ersetzt ist, Y = 1, 2, 3 oder 4, vorzugsweise 0 und 1 bedeutet und die übrigen der Substituenten $R^6$, $R^7$, $R^8$, $R^9$ und $R^{10}$ wie vorstehend definiert sind.

**6.** Verfahren zur Herstellung einer Verbindung nach den Ansprüchen 1 bis 5, dadurch gekennzeichnet ist, daß man
eine Verbindung der Formel IV

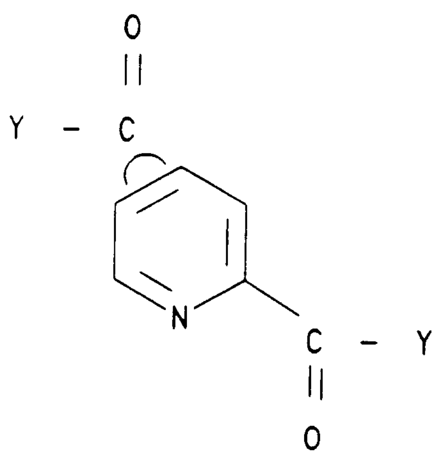

( I V )

mit einer Verbindung der Formeln V

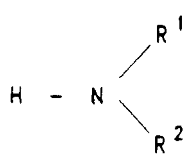

oder

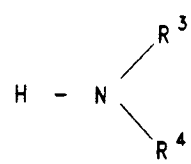

( V )

umsetzt, wobei $R^1$, $R^2$ bzw. $R^3$, $R^4$ die in den Ansprüchen 1 bis 5 angegebenen Bedeutungen haben und Y Halogen oder Hydroxy ist oder zusammen mit der Carbonylgruppe einen Aktivester oder ein gemischtes Anhydrid bildet, und die Reaktionsprodukte gegebenenfalls in ihre physiologisch verträgli-

chen Salze überführt.

7. Verbindungen nach einem der Ansprüche 1 bis 5 zur Inhibierung der Prolin- und Lysinhydroxylase.

8. Verbindungen nach den Ansprüchen 1 bis 5 zur Anwendung als Fibrosuppressiva und Immunsuppressiva.

9. Arzneimittel, enthaltend eine Verbindung der Formel I und einen verträglichen pharmazeutischen Träger.

10. Verwendung von Verbindung der Formel I zur Beeinflussung des Stoffwechsels von Collagen und collagenähnlichen Stoffen sowie der Biosynthese von $C1_q$,

11. Verwendung von Verbindungen der Formel I zur Behandlung von Störungen des Stoffwechsels von Collagen und collagenähnlichen Stoffen sowie der Biosynthese von $C1_q$.

12. Verfahren zur Herstellung von Arzneimitteln zur Beeiflussung des Stoffwechsels von Collagen und collagenähnlichen Stoffen sowie der Biosynthese von $C1_q$, dadurch gekennzeichnet, daß das Arzneimittel eine Verbindung der Formel I enthält.

Europäisches Patentamt

# EUROPÄISCHER RECHERCHENBERICHT

Nummer der Anmeldung

EP 92 11 8814

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| A | EP-A-0 438 795 (HOECHST AKTIENGESELLSCHAFT)<br>* das ganze Dokument *<br>--- | 1-12 | C07D213/81<br>C07D213/82<br>A61K31/44 |
| A<br><br><br>D | EP-A-0 409 119 (HOECHST AKTIENGESELLSCHAFT)<br>* das ganze Dokument *<br>& DE-A-3 924 093<br>--- | 1-12 | |
| A<br><br><br>D<br>D | EP-A-0 353 668 (HOECHST AKTIENGESELLSCHAFT)<br>* das ganze Dokument *<br>& DE-A-3 828 140<br>& DE-A-3 826 471<br>--- | 6 | |
| A<br><br><br>D | EP-A-0 278 453 (HOECHST AKTIENGESELLSCHAFT)<br>* das ganze Dokument *<br>& DE-A-3 703 959<br>----- | 1-12 | |

RECHERCHIERTE SACHGEBIETE (Int. Cl.5 )

C07D
A61K

Der vorliegende Recherchenbericht wurde für alle Patentansprüche erstellt

| Recherchenort | Abschlußdatum der Recherche | Prüfer |
|---|---|---|
| DEN HAAG | 19 JANUAR 1993 | P. BOSMA |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)